# EUROPEAN PATENT APPLICATION

(11) **EP 2 351 826 A1**
(43) Date of publication of application: **03.08.2011**
(21) Application number: 09823325.7
(22) Date of filing: 29.10.2009
(51) Int. Cl.: C12M 3/02

(54) **KIT FOR PREPARATION OF ANTIGEN-SPECIFIC CYTOTOXIC T LYMPHOCYTE**

(30) Priority: 31.10.2008 JP 2008280786; 15.07.2009 JP 2009166630
(71) Applicant: T-Cell Technologies, Inc., Nagoya, Aichi 460-0008 (JP); Medical and Biological Laboratories Co., Ltd., Nagoya, Aichi 460-0008 (JP); National University Corporation Nagoya University, Aichi 464-8601 (JP)
(72) Inventor: SUZUKI, Susumu, Kamiina-gun Nagano 399-4301 (JP); KOJIMA, Seiji, Nagoya-shi Aichi 464-8601 (JP); NAOE, Tomoki, Nagoya-shi Aichi 464-8601 (JP)
(74) Representative: Steinecke, Peter
(86) International application number: PCT/JP2009/005723
(87) International publication number: WO 2010/050212

(57) **Abstract**

The invention is intended to further improve the operability, economic efficiency and safety in the preparation of antigen-specific CTLs. The invention provides a preparation kit used for a method for preparing antigen-specific cytotoxic T lymphocytes, the method comprising: a first step for inducing antigen-specific cytotoxic T lymphocytes, wherein the components of the first step include a culture medium contained in an injection vessel, a hermetically sealed culture vessel, and the like; a second step for preparing an activated T cell for antigen presentation, wherein the components of the second step include a culture medium contained in an injection vessel, a hermetically sealed culture vessel, and the like.; and a third step for proliferating antigen-specific cytotoxic T lymphocytes, wherein the components of the third step include a culture medium contained in an injection vessel, a hermetically sealed separation vessel, a hermetically sealed culture vessel, and the like.

## Description

### TECHNICAL FIELD

The present invention relates to a preparation kit used for preparing an antigen-specific cytotoxic T lymphocyte (hereinafter also referred to as "CTL") and use thereof. The present application claims priorities of Japanese patent application No. 2008-280786, filed on October 31, 2008, and Japanese patent application No. 2009-166630, filed on July 15, 2009, the whole contents of which are incorporated herein by reference in their entirety.

### BACKGROUND ART

A therapy with an antigen-specific cytotoxic T lymphocyte (CTL therapy) is expected as an immunotherapy of the next generation. In the CTL therapy, antigen-specific CTLs are prepared ex vivo. Since complex and complicated operations have been accompanied so far in preparing such antigen-specific CTLs, we could not but rely on an open culture system. Therefore, facilities to keep a clean environment of a class 100 level had to be maintained and managed within a medical grade cell processing center (CPC), and a great expense was needed. In addition, since various kinds of cytokines are used in the conventional method, a considerable cost is required at the time of the preparation of such cytokines. Moreover, even though such preparation is performed in facilities where a clean environment of a class 100 level is maintained, culture in an open system is accompanied by a risk of safety threats. In this way, operability, economic efficiency and safety were a big obstruction to practical use in the conventional method for preparing antigen-specific CTLs and such a method was possible only in limited facilities.

In addition, one of the present applicants proposed a virus-specific CTL culture system which was simple and safe, as well as operable at low cost, in the previous patent application (patent document 1). The culture system concerned is characterized mainly in that dendritic cells are not used in the induction and proliferation of antigen-specific cytotoxic T lymphocytes and that a CD137 antigen is used in the isolation of the induced CTLs.

### PRIOR ART DOCUMENTS

### PATENT DOCUMENTS

Patent document 1: International Publication No. WO 2008/023786 Pamphlet

### SUMMARY OF THE INVENTION

### PROBLEMS TO BE SOLVED BY THE INVENTION

The culture system suggested by patent document 1 has brought a dramatic progress when compared to the conventional systems. However, any special means/techniques have not been adopted with respect to how to treat cells in each step, and there is a room for improvement in the operability. In addition, the importance of culturing not in an open system but in a closed system has been pointed out, but its concrete measures have not been provided.
Therefore, the present invention is intended to further improve the operability, economic efficiency and safety in the preparation of antigen-specific CTLs and promote the practical use of the CTL therapy.

### MEANS FOR SOLVING THE PROBLEMS

Based on the culture system reported in the previous patent application (patent document 1), the present applicants have studied extensively in order to solve the above-mentioned problem. As a result, they have found kit components which are extremely useful for the preparation of antigen-specific cytotoxic T lymphocytes by reviewing the each step and by applying an original device to the culture medium, form of culture vessel or the like, and handling method, etc. In this way, the present invention has been completed as a result of intensive investigations by the present inventors, and is given as follows.
[1] A preparation kit used for a method for preparing an antigen-specific cytotoxic T lymphocyte, the method comprising: a first step wherein an antigen-specific cytotoxic T lymphocyte is induced; a second step wherein an activated T cell for antigen presentation is prepared; and a third step wherein the antigen-specific cytotoxic T lymphocyte is proliferated using the antigen-specific cytotoxic T lymphocyte induced in the first step and the activated T cell for antigen presentation prepared in the second step;
   the components in the first step including:
   (1-1) an antigen peptide-containing culture medium contained in an injection vessel,
   (1-2) at least two culture media, each containing IL-2 contained in an injection vessel, and
   (1-3) a hermetically sealed culture vessel including a first port to inject a peripheral blood mononuclear cell that is separately prepared, a second port to inject the antigen peptide-containing culture medium, a third port(s) to inject the antigen peptide-containing culture medium wherein the number of the ports is at least the same as the number of the antigen peptide-containing culture media, and a fourth port to export the induced antigen-specific cytotoxic T lymphocyte,
   the components in the second step including:
   (2-1) an anti-CD3 antibody-containing culture medium contained in an injection vessel,
   (2-2) at least two culture media each containing IL-2 contained in an injection vessel,
   (2-3) an antigen peptide-containing culture medium contained in an injection vessel, and
   (2-4) a hermetically sealed culture vessel including a first port to inject a peripheral blood mononuclear cell that is separately prepared, a second port to inject the anti-CD3 antibody-containing culture medium, a third port(s) to inject the IL-2-containing culture medium wherein the number of the ports is at least the same as the number of the IL-2-containing culture media, a fourth port to inject the antigen peptide-containing culture medium , and a fifth port to export the activated T cell for antigen presentation that has been prepared, and
   the components in the third step including:
   (3-1) a separation medium for antigen-specific cytotoxic T lymphocytes contained in an injection vessel,
   (3-2) a separation medium for activated T cells for antigen presentation contained in an injection vessel,
   (3-3) a first hermetically sealed separation vessel including a first port to import the induced antigen-specific cytotoxic T lymphocytes, a second port to drain waste fluid, a third port to inject the separation medium for antigen-specific cytotoxic T lymphocytes, and a fourth port to export the antigen-specific cytotoxic T lymphocyte after separation,
   (3-4) a second hermetically sealed separation vessel including a first port to import the activated T cells for antigen presentation that has been prepared, a second port to drain waste fluid, a third port to inject a separation medium for the activated T cells for antigen presentation, and a fourth port to export the activated T cell for antigen presentation,
   (3-5) at least two proliferation culture media, each containing IL-2 or IL-15, or both of them, contained in an injection vessel, and
   (3-6) a hermetically sealed culture vessel including a first port to import an antigen-specific cytotoxic T lymphocyte after separation, a second port to import a cell for antigen presentation after separation, a third port to inject serum or plasma that is separately prepared, a fourth port(s) to inject the proliferation culture medium wherein the number of the ports is at least the same as the number of the proliferation culture media, and a fifth port to export the proliferated antigen-specific cytotoxic T lymphocyte.
[2] The preparation kit according to [1], wherein the number of the IL-2-containing culture media in the above (1-2) is 3 to 5.
[3] The preparation kit according to [1] or [2], wherein the number of the IL-2-containing culture media in the above (2-2) is 3 to 6.
[4] The preparation kit according to any one of [1] to [3], wherein the separation medium for antigen-specific cytotoxic T lymphocyte in the above (3-1) and the separation medium for activated T cells for antigen presentation in the above (3-2) are each an IL-2-containing culture medium.
[5] The preparation kit according to any one of claims 1 to 4, wherein two kinds of caps are provided at the first to third ports of the hermetically sealed culture vessel in the above (1-3), the first to fourth ports of the hermetically sealed culture vessel in the above (2-4), and the third and fourth ports of the hermetically sealed culture vessel in the above (3-6), respectively, the two kinds of caps being comprised of a cap attached when not in use and a cap that is distinguishable from the cap and attached after use.
[6] The preparation kit according to any one of [1] to [5], wherein the third port of the hermetically sealed culture vessel in the above (1-3), the third port of the hermetically sealed culture vessel in the above (2-4), and the fourth port of the hermetically sealed culture vessel in the above (3-6) are each a branched port.
[7] The preparation kit according to any one of [1] to [6], wherein each of the hermetically sealed culture vessel in the above (1-3), the hermetically sealed culture vessel in the above (2-4), and the hermetically sealed culture vessel in the above (3-6) includes a spare port.
[8] The preparation kit according to any one of [1] to [7], wherein a culture medium for peripheral blood mononuclear cells contained in a vessel is further included.
[9] The preparation kit according to [8], wherein a vessel for preparing peripheral blood mononuclear cells is further included.
[10] The preparation kit according to any one of [1] to [9], wherein a separation vessel for separating the antigen-specific cytotoxic T lymphocytes that have been proliferated in the third step is further included.
[11] The preparation kit according to [10], wherein a preservation vessel to cryopreserve the cells that have been separated using the separation vessel is further included.
[12] A preparation kit used for a method for preparing an antigen-specific cytotoxic T lymphocyte, the method comprising: a first step wherein an antigen-specific cytotoxic T lymphocyte is induced; a second step wherein an antigen-presenting cell is prepared; and a third step wherein the antigen-specific cytotoxic T lymphocyte is proliferated using the antigen-specific cytotoxic T lymphocyte induced in the first step and the antigen-presenting cell prepared in the second step;
   the components in the first step including:
   (1-1) an antigen peptide-containing culture medium contained in an injection vessel,
   (1-2) at least two culture media each containing IL-2, contained in an injection vessel, and
   (1-3) a hermetically sealed culture vessel including a first port to inject a peripheral blood mononuclear cell that is separately prepared, a second port to inject the antigen peptide-containing culture medium, a third port(s) to inject the antigen peptide-containing culture medium wherein the number of the ports is at least the same as the number of the antigen peptide-containing culture media, and a fourth port to export the induced antigen-specific cytotoxic T lymphocyte,
   the components in the second step including:
   an antigen-presenting cell in a freeze-dried state contained in a vessel and
   the components in the third step including:
      (3-1) a separation medium for antigen-specific cytotoxic T lymphocytes, contained in an injection vessel,
      (3-2) a first hermetically sealed separation vessel including a first port to import the induced antigen-specific cytotoxic T lymphocyte, a second port to drain waste fluid, a third port to inject a separation medium for the antigen-specific cytotoxic T lymphocytes, and a fourth port to export an antigen-specific cytotoxic T lymphocyte after separation,
      (3-3) at least two proliferation culture media, each containing IL-2 or IL-15, or both of them in an injection vessel, and
      (3-4) a hermetically sealed culture vessel including a first port to import an antigen-specific cytotoxic T lymphocyte after separation, a second port to import the prepared antigen-presenting cell, a third port to inject serum or plasma that is separately prepared, a fourth port(s) to inject the proliferation culture medium wherein the number of the ports is at least the same as the number of the proliferation culture media, and a fifth port to export the proliferated antigen-specific cytotoxic T lymphocyte.
[13] A method for preparing an antigen-specific cytotoxic T lymphocyte, which comprises using a preparation kit according to any one of [1] to [12].

### EFFECT OF THE INVENTION

According to the preparation kit of the invention, an antigen-specific cytotoxic T lymphocyte can be prepared by a simple and easy operation. In addition, the time required for the preparation can be significantly shortened. On the other hand, all culture steps for the preparation kit of the invention can be performed in a closed system, thereby to enhance the safety. The requirement level for facilities may be lowered by ensuring a high safety, and thus antigen-specific cytotoxic T lymphocytes can be prepared at a low cost.

### BRIEF DESCRIPTION OF THE DRAWINGS

Fig. 1 shows an example of the preparation kit of antigen-specific CTLs. Among the kit components, the components to be used in the induction step of antigen-specific CTLs are shown.
Fig. 2 shows an example of the preparation kit of antigen-specific CTLs. Among the kit components, the components to be used in the preparation step of antigen-presenting cells are shown.
Fig. 3 shows an example of the preparation kit of antigen-specific CTLs. Among the kit components, the components to be used in the proliferation step of antigen-specific CTLs are shown.
Fig. 4 shows an example of the preparation kit of antigen-specific CTLs. Among the kit components, the components to be used in the proliferation step of antigen-specific CTLs are shown.
Fig. 5 is a drawing showing a tube to import/export cells and a tube to dispense cells.
Fig. 6 is a flow chart showing the induction step of antigen-specific CTLs.
Fig. 7 is a flow chart showing the preparation step of antigen-presenting cells.
Fig. 8 is a flow chart showing the proliferation step of antigen-specific CTLs.
Fig. 9 is a flow chart showing each step after the proliferation step of antigen-specific CTLs.
Fig. 10 shows the results of flow cytometry analysis. Samples were taken on days 0, 7, and 14 as shown in Fig. 6 (a flow chart of the induction step of antigen-specific CTLs) and on day 21 as shown in Fig. 8 (a flow chart showing the proliferation step of antigen-specific CTLs), and then analyzed by flow cytometry (upper part). The lower part is a graph showing changes with time of the total cell count and the tetramer-positive cell count.

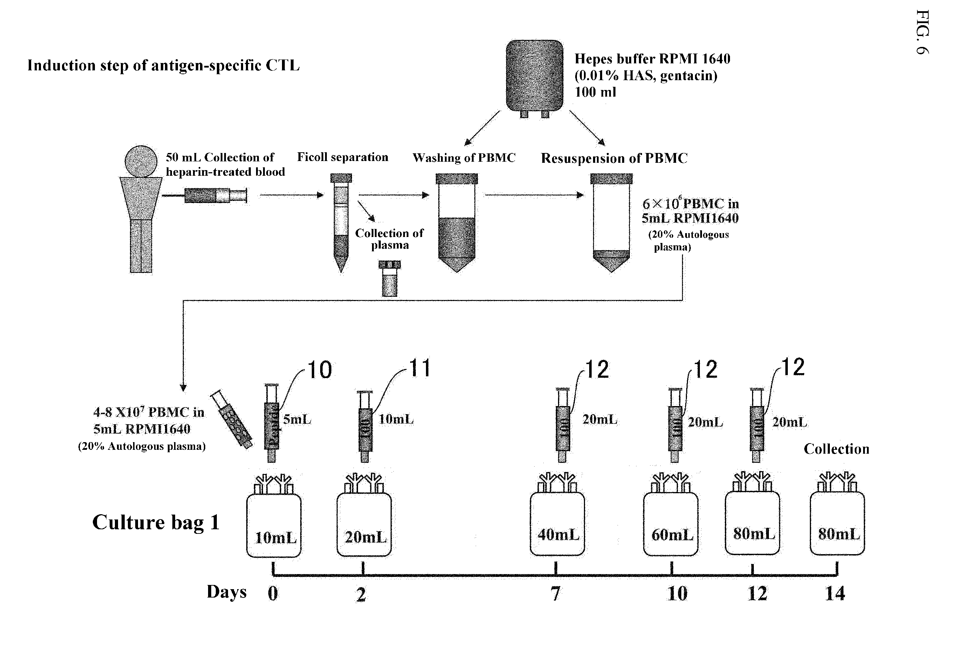

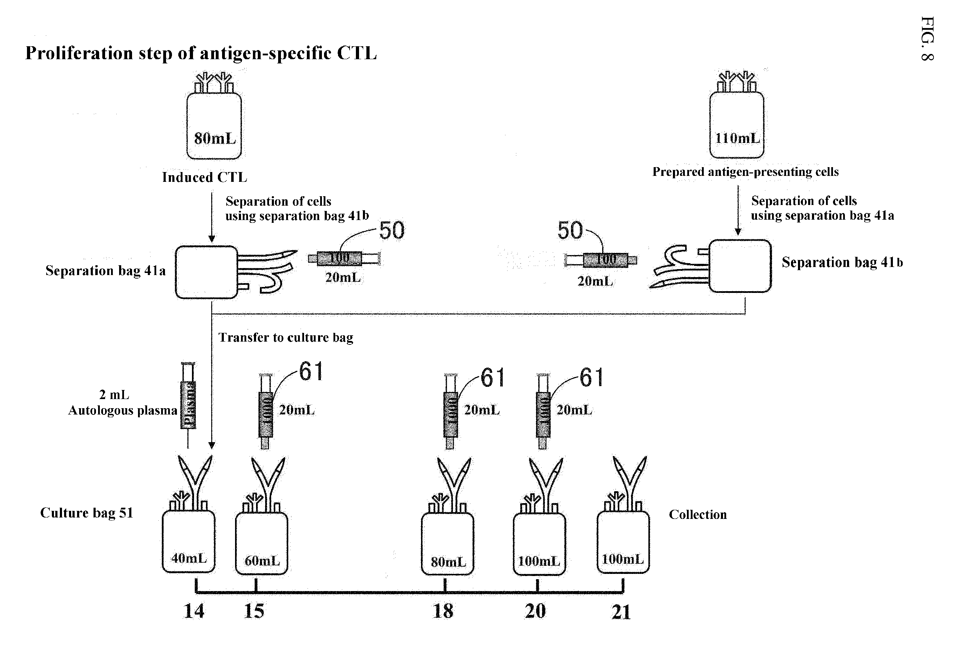

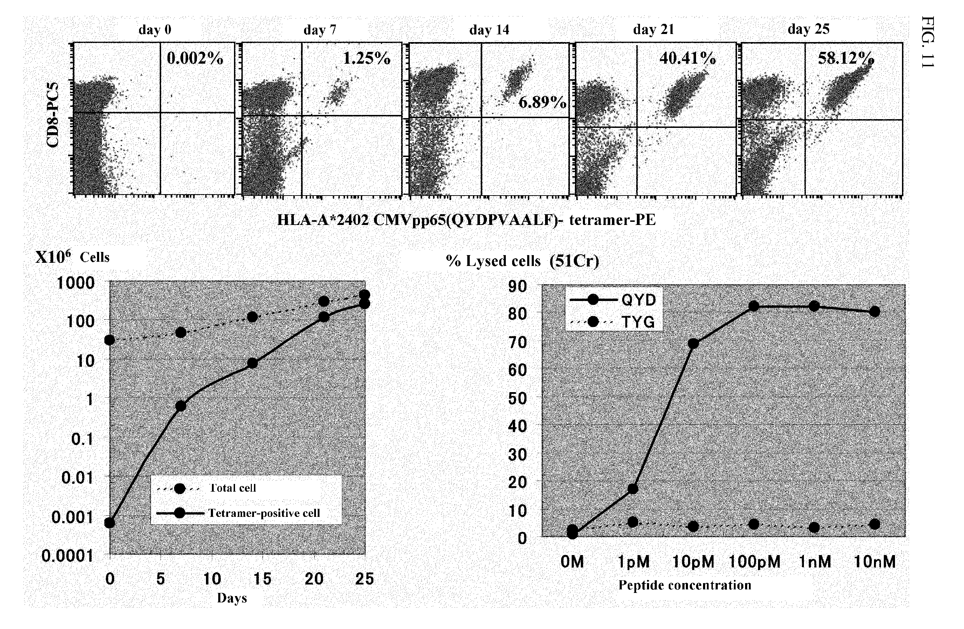

Fig. 11 shows the results of flow cytometry analysis. Samples were taken on days 0, 7, and 14 as shown in Fig. 6 (a flow chart of the induction step of antigen-specific CTLs) and on day 21 and on the day after additional 4-day culture (on day 25) as shown in Fig. 8 (a flow chart showing the proliferation step of antigen-specific CTLs), and then analyzed by flow cytometry (upper part). The lower left part is a graph showing changes with time of the total cell count and the tetramer-positive cell count. The lower right part is a graph showing the results of the cytotoxic activity test. In the cytotoxic activity test, EBV (Epstein-Barr virus)-infected LCL (lymphoblastoid cells) prepared from a donor was served as a target cell, this cell was mixed with an effector cell (tetramer-positive cell) in a predetermined ratio (effector cell/target cell=10), and after addition of an antigen peptide at a respective concentration, the resulting mixture was incubated. The cytotoxic activity was evaluated from the amount of ⁵¹Cr detected with the lysis of the cells. QYD (HLA-A*2402 restricted CMV pp65 epitope peptide) and TYG (HLA-A*2402 restricted EBV LMP2a epitope peptide) (control) were used as antigen peptides.
Fig. 12 shows the results of flow cytometry analysis. Samples were taken on days 0, 7, and 14 as shown in Fig. 6 (a flow chart of the induction step of antigen-specific CTLs) and on day 21 and on the day after additional 3-day culture (on day 24) as shown in Fig. 8 (a flow chart showing the proliferation step of antigen-specific CTLs), and then analyzed by flow cytometry (upper part). The lower part is a graph showing changes with time of the total cell count and the tetramer-positive cell count.

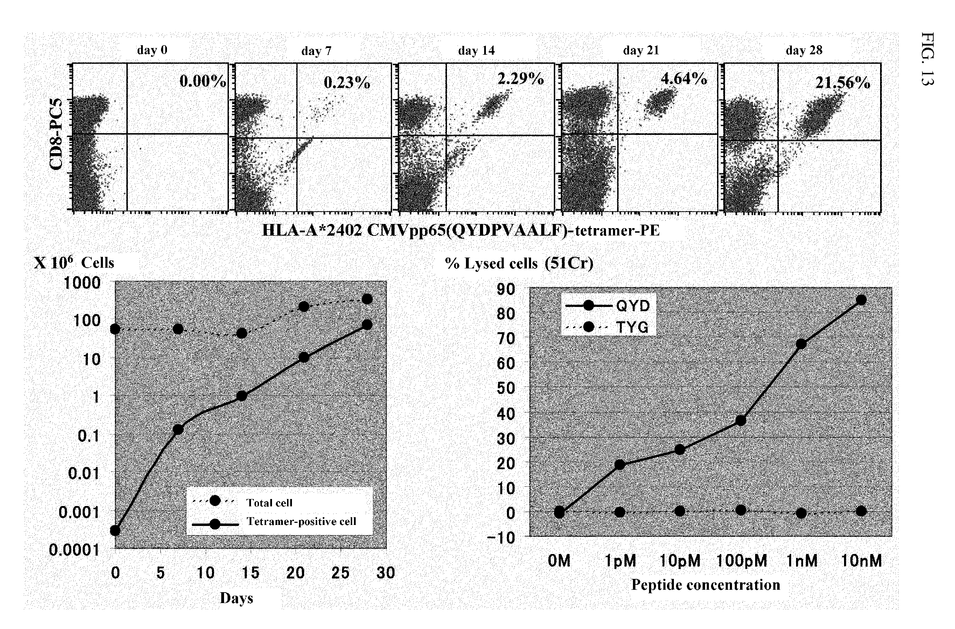

Fig. 13 shows the results of flow cytometry analysis. Samples were taken on days 0, 7, and 14 as shown in Fig. 6 (a flow chart of the induction step of antigen-specific CTLs) and on day 21 and on the day after additional 7-day culture (on day 28) as shown in Fig. 8 (a flow chart showing the proliferation step of antigen-specific CTLs), and then analyzed by flow cytometry (upper part). The lower left part is a graph showing changes with time of the total cell count and the tetramer-positive cell count. The lower right part is a graph showing the results of the cytotoxic activity test. In the cytotoxic activity test, EBV (Epstein-Barr virus)-infected LCL (lymphoblastoid cells) prepared from a donor was served as a target cell, this cell was mixed with an effector cell (tetramer-positive cell) in a predetermined ratio (effector cell/target cell=10), and after addition of an antigen peptide at a respective concentration, the resulting mixture was incubated. The cytotoxic activity was evaluated from the amount of ⁵¹Cr detected with the lysis of the cells. QYD (HLA-A*2402 restricted CMV pp65 epitope peptide) and TYG (HLA-A*2402 restricted EBV LMP2a epitope peptide) (control) were used as antigen peptides.

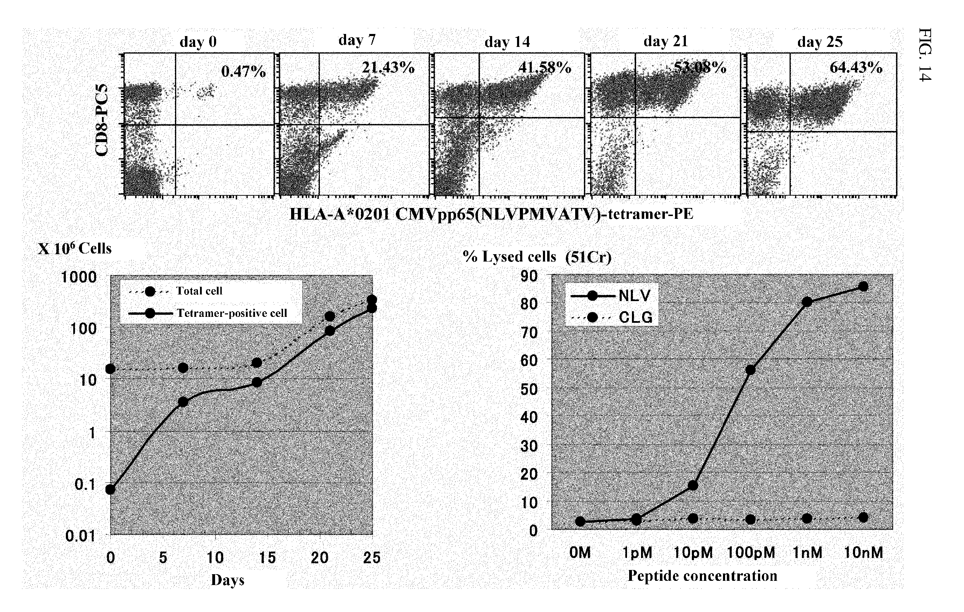

Fig. 14 shows the results of flow cytometry analysis. Samples were taken on days 0, 7, and 14 as shown in Fig. 6 (a flow chart of the induction step of antigen-specific CTLs) and on day 21 and on the day after additional 4-day culture (on day 25) as shown in Fig. 8 (a flow chart showing the proliferation step of antigen-specific CTLs), and then analyzed by flow cytometry (upper part). The lower left part is a graph showing changes with time of the total cell count and the tetramer-positive cell count. The lower right part is a graph showing the results of the cytotoxic activity test. In the cytotoxic activity test, EBV (Epstein-Barr virus)-infected LCL (lymphoblastoid cells) prepared from a donor was served as a target cell, this cell was mixed with an effector cell (tetramer-positive cell) in a predetermined ratio (effector cell/target cell=10), and after addition of an antigen peptide at a respective concentration, the resulting mixture was incubated. The cytotoxic activity was evaluated from the amount of ⁵¹Cr detected with the lysis of the cells. NLV (HLA-A*0201 restricted CMV pp65 epitope peptide) and CLG (HLA-A*0201 restricted EBV LMP2a epitope peptide)(control) were used as antigen peptides.
Fig. 15 shows an example of the preparation kit of antigen-specific CTLs utilizing antigen-presenting cells in a freeze-dried state. Among the kit components, the components to be used in the induction step of antigen-specific CTLs are shown.
Fig. 16 shows an example of the preparation kit of antigen-specific CTLs utilizing antigen-presenting cells in a freeze-dried state. Among the kit components, the components to be used in the preparation step of antigen-presenting cells are shown.
Fig. 17 shows an example of the preparation kit of antigen-specific CTLs utilizing antigen-presenting cells in a freeze-dried state. Among the kit components, the components to be used in the proliferation step of antigen-specific CTLs are shown.
Fig. 18 shows an example of the preparation kit of antigen-specific CTLs utilizing antigen-presenting cells in a freeze-dried state. Among the kit components, the components to be used in the proliferation step of antigen-specific CTLs are shown.
Fig. 19 shows an example of the conditions for freeze-drying antigen-presenting cells.

### MODE FOR CARRYING OUT THE INVENTION

A first aspect of the invention relates to a preparation kit used for preparing an antigen-specific cytotoxic T lymphocyte (antigen-specific CTL). The "antigen-specific CTL" means a CTL having a high specificity to a specific antigen. The antigen-specific CTL specifically recognizes a cell that expresses or presents a corresponding antigen, and shows a cytotoxic activity. Although an antigen-specific CTL is induced in vivo by the defense mechanism in the living body, the "antigen-specific CTL" is induced ex vivo in the present invention.

The preparation kit of the invention includes the components that are necessary to carry out an induction of antigen-specific CTLs (first step), preparation of activated T cells for antigen presentation (second step), and proliferation of antigen-specific CTLs (third step). The components of each step included in the preparation kit of the invention will be explained.

### (First Step: Induction of CTL)

In this step, a specific CTL against a specific antigen is induced by a peripheral blood mononuclear cell. For carrying out the step concerned, the preparation kit of the invention includes at least the following components (1-1) to (1-3):
(1-1) an antigen peptide-containing culture medium contained in an injection vessel,
(1-2) at least two culture media, each containing IL-2 contained in an injection vessel, and
(1-3) a hermetically sealed culture vessel including a first port to inject peripheral blood mononuclear cells that are separately prepared, a second port to inject the antigen peptide-containing culture medium, a third port(s) to inject the IL-2-containing culture medium wherein the number of the ports is at least the same as the number of the IL-2-containing culture media, and a fourth port to export the induced antigen-specific cytotoxic T lymphocytes.

Hereinafter, details of each component will be explained. In addition, in the following explanation, "an antigen peptide-containing culture medium" means "an antigen peptide-containing culture medium contained in an injection vessel" unless otherwise indicated. Similarly, "an IL-2-containing culture medium" means "an IL-2-containing culture medium contained in an injection vessel".

### (1-1) Antigen Peptide-Containing Culture Medium

The form of the injection vessel is not particularly limited as long as the medium can be maintained, and the medium to be contained can be injected into the following hermetically sealed culture vessel. For example, a hermetically sealed injector (a syringe) may be used as an injection vessel.

An antigen peptide-containing culture medium is contained in the injection vessel. The antigen peptide-containing culture medium is a culture medium in which an antigen peptide is contained according to the purposes. Examples of the antigen peptide are HLA-binding peptides of viruses (cytomegalovirus, Epstein-Barr virus, adenovirus, etc.) or of tumor antigens (WT1, hTERT, MN/CA9, gp100, MART1, TRP1, TRP2, tyrosinase, MAGE1, MAGE2, MAGE3, MAGE6, NY-ESO-1, MUM1, BAGE, GAGE1, GAGE2, CEA, PSA, etc.). Only one kind of antigen peptide is usually used, but use of two or more antigen peptides are not precluded. The antigen peptide-containing culture medium can be prepared by adding an antigen peptide to a culture medium suitable for the culture of T cells (e.g., RPMI 1640, AIM-V, Dulbecco's modified eagle medium, etc.). Serum, plasma, serum albumin, antibiotic, L-glutamine, and the like may be added thereto. The amount of the antigen peptide in the culture medium is, for example, 0.01 µg/mL to 100 µg/mL. The amount of the antigen peptide-containing culture medium should be, for example, 5 mL to 50 mL. The size of the injection vessel may be determined depending on the amount of the antigen peptide-containing culture medium. For example, when an antigen peptide-containing culture medium of 5 mL is used, a syringe for 5 mL may be adopted as an injection vessel.

### (1-2) IL-2-Containing Culture Medium

Explanation of the constitution of the injection vessel is omitted because it is similar to the case of (1-1). An IL-2-containing culture medium is contained in the injection vessel. The IL-2-containing culture medium can be prepared by adding IL-2 to a culture medium suitable for the culture of T cells (e.g., RPMI 1640, AIM-V, Dulbecco's modified eagle medium, X-VIVO (BioWhittaker Inc.), ALyS505N (Cell Science & Technology Institute, Inc.)). Serum, plasma, serum albumin, antibiotic, L-glutamine, and the like may be added thereto. It is preferable to use a recombinant human IL-2 (e.g. PROLEUKIN manufactured by Chiron company) as an IL-2. The content of the IL-2 is, for example, 10 IU/mL to 1,000 IU/mL.

In the present invention, at least two IL-2- containing culture media, each being contained in an injection vessel, are used. Preferably, the number of the IL-2-containing culture media should be 3 or more. Specifically, the number of the IL-2-containing culture media should be 3 to 5. If the number of IL-2-containing culture media is increased, the number of times of adding a culture medium in the induction step of CTLs can be increased. As a result, an improvement in the induction efficiency and/or an increase in the number of the induced cells is attained.

With respect to all IL-2-containing culture media, it is not necessary to unify the culture medium composition (IL-2 content or other ingredient contents) and the liquid amount. For example, various combinations are possible, such as a combination of an IL-2-containing culture medium having a little amount of liquid (e.g., 10 mL) with an IL-2-containing culture medium having a large amount of liquid (e.g., 20 mL), or a combination of an IL-2-containing culture medium having a little amount of IL-2 (e.g., 100 IU/mL) with an IL-2-containing culture medium having a large amount of IL-2 (e.g., 1,000 IU/mL). In the case of the combination of the former, the number of the IL-2-containing culture media having a little amount of liquid should be preferably 1 and the number of the IL-2-containing culture media having a large amount of liquid should be preferably 2 to 4 in consideration of operability and efficiency. In addition, a part of the IL-2-containing culture media may be used as a spare medium.

### (1-3) Hermetically Sealed Culture Vessel

The hermetically sealed culture vessel comprises at least four kinds of ports (the first to fourth ports). The first port is a port to inject peripheral blood mononuclear cells that are separately prepared. Similarly, the second port is a port to inject an antigen peptide-containing culture medium, the third port is a port to inject an IL-2-containing culture medium, and the fourth port is a port to export the induced antigen-specific cytotoxic T lymphocytes.

The second port is provided in at least the same number as the number of the IL-2-containing culture media. In other words, the number of the second ports should be equal to or greater than the number of the IL-2-containing culture media. When the second ports with the number greater than the number of IL-2-containing culture media are provided, a part of such second ports can be used as a spare port. The numbers of the first ports, the third ports, and the fourth ports are all 1 as a general rule. However, a spare port may be provided in order to cope with an operation error. The spare ports can be provided for each port, or can be provided as a common port for two or more ports (e.g., the second port and the third port).

The form of each port is not particularly limited. For example, any forms such as luer lock type, puncture port, and film tube, etc. may be adopted. Above all, it is preferable to adopt a luer lock type port. This is because it can prevent a liquid leak by certain fixation. All ports need not be of the same form. For example, the first port can be a port wherein the injection with a needle is possible (e.g. a port having an injection mouth made of an elastomer such as rubber, etc.) and the other ports can be of a luer lock type. In one preferable embodiment, all ports are of a luer lock type. According to the constitution concerned, a liquid leak can be prevented in each injection operation, and operability is improved because operation methods are in common.

A part or all of the first to fourth ports may be a branched port. The number of joints between the port and the main body of the vessel decreases when the branched port is adopted, thereby to simplify the structure. Therefore, production and handling of the ports become easy, and also the occurrence of the malfunction when in use can be prevented. Thus, in the present invention wherein a considerable number of ports should be provided in a hermetically sealed vessel, use of such a branched port is an advantageous constitution.

The number of branches in the branched port is not particularly limited. For example, a branched port with 2 to 4 branches can be used. Preferably, at least the third port for an IL-2-containing culture medium (1-2) should be a branched port. In addition, in the present specification, the number of branches is considered as the number of ports in the case of branched ports. For example, 3-branched port is considered as three ports.

A special constitution in the hermetically sealed culture vessel of the invention resides in each port. Therefore, the constitution of the hermetically sealed culture vessel of the invention should follow a well-known constitution (for example, various bags which are used in cell culture) except for a part involved in each port. For reasons of easy handling and the like, it is preferable to take a form of bag made of flexible materials.

### (Second Step: Preparation of Activated T Cells for Antigen Presentation)

In this step, an activated T cell for antigen presentation was prepared from peripheral blood mononuclear cells. For carrying out the step concerned, the preparation kit of the invention includes at least the following components (2-1) to (2-4). In addition, the "activated T cell for antigen presentation" is a cell that expresses a co-stimulator on the cell surface by adding a specific stimulation to a peripheral blood mononuclear cell, and functions as an antigen-presenting cell to promote the proliferation of antigen-specific CTLs. When the preparation kit of the invention is used, an activated T cell for antigen presentation is prepared by using an anti-CD3 antibody. In addition, for convenience of explanation, "an activated T cell for antigen presentation" is hereinafter abbreviated as "an antigen-presenting cell".
(2-1) An anti-CD3 antibody-containing culture medium contained in an injection vessel.
(2-2) At least two culture media each containing IL-2 contained in an injection vessel.
(2-3) An antigen peptide-containing culture medium contained in an injection vessel.
(2-4) A hermetically sealed culture vessel including a first port to inject peripheral blood mononuclear cells that are separately prepared, a second port to inject the anti-CD3 antibody-containing culture medium, a third port(s) to inject the IL-2-containing culture medium wherein the number of the ports is at least the same as the number of the IL-2-containing culture media, a fourth port to inject the antigen peptide-containing culture medium, and a fifth port to export the prepared activated T cells for antigen presentation.

Details of each component will be given below, but the same explanation of the matters that are not particularly referred to (constitution of the injection vessel and the constitution of the hermetically sealed culture vessel except for the part involved in the port) as in the first step is quoted because it is the same as in the corresponding components of the first step. In addition, in the following explanation, "an anti-CD3 antibody-containing culture medium" means "an anti-CD3 antibody-containing culture medium contained in an injection vessel" unless otherwise indicated. Similarly, "an IL-2-containing culture medium" means "an IL-2-containing culture medium contained in an injection vessel", and "an antigen peptide-containing culture medium" means "an antigen peptide-containing culture medium contained in an injection vessel".

### (2-1) Anti-CD3 Antibody-Containing Culture Medium

The anti-CD3 antibody used for the anti-CD3 antibody-containing culture medium can be prepared by the immunological technique using a CD3 molecule or its part. A commercially available anti-CD3 antibody also can be used. Example of the commercially available anti-CD3 antibody is OKT-3 antibody (Janssen Pharmaceutical K.K.). It is preferable to use OKT-3 antibody to which a drug approval has been granted, in view of safety. In addition, the anti-CD3 antibody may be a polyclonal antibody or a monoclonal antibody. However, it is preferable to use a monoclonal anti-CD3 antibody in consideration of specificity and efficiency. It is also possible to use two or more kinds of anti-CD3 antibodies together.

The anti-CD3 antibody-containing culture medium can be prepared by adding an anti-CD3 antibody to a culture medium suitable for the culture of T cells (e.g., RPMI 1640, AIM-V, Dulbecco's modified eagle medium, etc.). Serum, plasma, serum albumin, antibiotic, L-glutamine, and the like may be added thereto. The amount of the anti-CD3 antibody in the culture medium is, for example, 0.01 µg/mL to 10 µg/mL. The amount of the anti-CD3 antibody-containing culture medium should be, for example, 5 mL to 50 mL.

### (2-2) IL-2-Containing Culture Medium

Two or more IL-2-containing culture media are used similarly as in the IL-2-containing culture media of (1-2). Preferably, the number of the IL-2-containing culture media should be 3 or more. More preferably, the number of the IL-2-containing culture media should be 4 or more. Specifically, the number of the IL-2-containing culture media should be, for example, 3 to 6. If the number of IL-2-containing culture media is increased, the number of times of adding a culture medium in the preparation step of antigen-presenting cells can be increased. As a result, an improvement of the preparation efficiency and/or an increase of the number of the cells to be prepared is attained.

With respect to all IL-2-containing culture media, it is not necessary to unify the culture medium composition (IL-2 content or other ingredient contents) and liquid amount. For example, various combinations are possible, such as a combination of an IL-2-containing culture medium having a little amount of liquid (e.g., 10 mL) with an IL-2-containing culture medium having a large amount of liquid (e.g., 20 mL), or a combination of an IL-2-containing culture medium having a little amount of IL-2 (e.g., 100 IU/mL) with an IL-2-containing culture medium having a large amount of IL-2 (e.g., 1,000 IU/mL). Preferably, an IL-2-containing culture medium wherein the IL-2 content and the liquid amount are both little (for example, liquid amount of 10 mL and IL-2 content of 100 IU/mL) and an IL-2-containing culture medium wherein the IL-2-content and the liquid amount are both large (for example, liquid amount of 20 mL and IL-2 content of 1000 IU/mL) are combined for use. In this case, the number of the former should be preferably 1 and the number of the latter should be preferably 3 to 5 in consideration of operability and effiency. In addition, a part of the IL-2-containing culture media may be used as a spare medium.

### (2-3) Antigen Peptide-Containing Culture Medium

The explanation about the antigen peptide-containing culture medium concerned is omitted because it is similar to the antigen peptide-containing culture medium of (1-1).

### (2-4) Hermetically Sealed Culture Vessel

This hermetically sealed culture vessel includes at least five kinds of ports (the first to fifth ports). The first port is a port to inject peripheral blood mononuclear cells that are separately prepared. Similarly, the second port is a port to inject an anti-CD3 antibody-containing culture medium, the third port is a port to inject an IL-2-containing culture medium, the fourth port is a port to inject an antigen peptide-containing culture medium, and the fifth port is a port to export the prepared activated T cells for antigen presentation.

The third port is provided in at least the same number as that of the IL-2-containing culture medium. In other words, the number of the second ports should be equal to or greater than the number of the IL-2-containing culture media. When the second ports with the number more than the number of IL-2-containing culture media are provided, a part of such ports can be used as a spare port. The numbers of the first ports, the third ports, the fourth ports, and the fifth ports are all 1 as a general rule. However, a spare port may be provided in order to cope with an operation error. The spare ports can be provided for each port, or can be provided as a common port for two or more ports (e.g., the second port and the third port).

The form or the like of each port is not particularly limited as in the hermetically sealed culture vessel of (1-3). As an example of the constitution of each port, the first port can be a port wherein the injection with a needle is possible (e.g. a port having an injection mouth made of an elastomer such as rubber, etc.) and the other ports can be of a luer lock type. Preferably, from the same reasons as in the hermetically sealed culture vessel of (1-3), all ports are of a luer lock type.

On the other hand, like in the case of the hermetically sealed culture vessel of (1-3), a part or all of the first to fifth ports may be a branched port. Preferably, at least the third port for the IL-2-containing culture medium should be a branched port.

### (Third Step: Proliferation of CTL)

This step comprises the proliferation of antigen specific CTL by using the antigen-specific CTL induced in the first step and the antigen-presenting cell prepared in the second step. For carrying out the step concerned, the preparation kit of the invention includes at least the following components (3-1) to (3-6):
(3-1) a separation medium for antigen-specific cytotoxic T lymphocytes contained in an injection vessel,
(3-2) a separation medium for cells for antigen presentation contained in an injection vessel,
(3-3) a first hermetically sealed separation vessel including a first port to import the induced antigen-specific cytotoxic T lymphocytes, a second port to drain waste fluid, a third port to inject a separation medium for the antigen-specific cytotoxic T lymphocytes, and a fourth port to export the antigen-specific cytotoxic T lymphocytes after separation,
(3-4) a second hermetically sealed separation vessel including a first port to import the prepared antigen-presenting cells, a second port to drain waste fluid, a third port to inject a separation medium for the antigen-presenting cells, and a fourth port to export the antigen-presenting cells after separation.
(3-5) at least two proliferation culture media contained in an injection vessel, each containing IL-2 or IL-15, or both of them, and
(3-6) a hermetically sealed culture vessel including a first port to import the antigen-specific cytotoxic T lymphocytes after separation, a second port to import the antigen-presenting cells after separation, a third port to inject serum or plasma that is separately prepared, a fourth port(s) to inject the proliferation culture medium wherein the number of the ports is at least the same as the number of the proliferation culture media, and a fifth port to export the proliferated antigen-specific cytotoxic T lymphocytes.

### (3-1) Separation Medium for Antigen-Specific CTLs and (3-2) Separation Medium for Antigen-Presenting Cells

The separation medium for antigen-specific CTLs and the separation medium for antigen-presenting cells are both those suitable for the culture of T cells (e.g., RPMI 1640, AIM-V, Dulbecco's modified eagle medium, X-VIVO (BioWhittaker Inc.) ALyS505N (Cell Science & Technology Institute, Inc.), etc.). It is preferable to use a culture medium supplemented with IL-2 or IL-15. It is also preferable to use a culture medium containing both IL-2 and IL-15. When IL-2 is added, the content is, for example, 10 IU/mL to 1,000 IU/mL. When IL-15 is added, the content is, for example, 10 ng/mL to 100 ng/mL. It is preferable to use a recombinant human IL-15 (e.g., CellGro IL-15 provided by CellGenix GmbH) as an IL-15. A culture medium supplemented with serum, plasma, serum albumin, antibiotic, L-glutamine, etc. may also be used. The compositions of the separation medium for antigen-specific CTLs and the separation medium for antigen-presenting cells are not necessarily the same as each other. Typically, one separation medium for antigen-specific CTLs and one separation medium for antigen-presenting cells are respectively provided, but in the case where washing step is performed at the time of separation as mentioned later, a necessary number of the culture media are to be prepared accordingly.

### (3-3) First Hermetically Sealed Separation Vessel

The first hermetically sealed separation vessel is used for separating the induced antigen-specific CTLs. The first hermetically sealed separation vessel includes at least four kinds of ports (the first to fourth ports). The first port is a port to import the induced antigen-specific CTLs. Similarly, a second port is a port to drain waste fluid, a third port is a port to inject a separation medium for antigen-specific cytotoxic T lymphocytes, and a fourth port is a port to export the CTLs after separation. There is no particular limitation to the form or the like of each port. For example, the third port is of a luer lock type.

The "separation" as used herein means the collection of cells from the cell-containing culture solution obtained in the first step. The "separation" in the invention comprises a series of operations including sedimentation (centrifugation) of the cells by centrifugal treatment, disposal of unnecessary culture medium (waste fluid), resuspension of the cells into a culture medium (injection of culture medium), and collection of the cell suspension (export of the cells). Without providing an exclusive port for waste fluid (the second port), the port to export the CTLs may also be used for draining waste fluid. Before the operation of the cell export, further centrifugal treatment, followed by drainage operation of waste fluid and injection operation of the culture medium, may be performed. In other words, a washing step for the cells may be incorporated. Also, such washing step may be repeated (for example, two to four times).

### (3-4) Second Hermetically Sealed Separation Vessel

The second hermetically sealed separation vessel is used for separating the prepared antigen-presenting cells. The second hermetically sealed separation vessel includes at least four kinds of ports (the first to fourth ports). The first port is a port to import the prepared antigen-presenting cells. Similarly, the second port is a port to drain waste fluid, the third port is a port to inject a separation medium for antigen-presenting cells, and the fourth port is a port to export the antigen-presenting cells after separation. The form or the like of each port is not particularly limited similarly as in the first hermetically sealed separation vessel. For example, the third port can be of a luer lock type. In addition, the explanation of "separation" used herein is omitted because it is similar to the case of the first hermetically sealed separation vessel.

### (3-5) Proliferation Culture Medium

Two or more proliferation culture media are provided. Preferably, the number of the culture media concerned should be 3 or more. Specifically, the number of the culture media should be 5 to 7. If the number of the culture media is increased, the number of times of adding a culture medium in the proliferation step of CTLs can be increased. As a result, an improvement in the proliferation efficiency and/or an increase in the number of the cells to be collected is attained. The proliferation culture medium contains IL-2 or IL-15, or both of them. The IL-2 content is, for example, 10 IU/mL to 1,000 IU/mL. On the other hand, the IL-15 content is, for example, in a range of 10 ng/mL to 100 ng/mL.

### (3-6) Hermetically Sealed Culture Vessel

This hermetically sealed culture vessel comprises at least five kinds of ports (the first to fifth ports). The first port is a port to import an antigen-specific CTL after separation. Similarly, the second port is a port to import an antigen-presenting cell after separation, the third port is a port to inject serum or plasma that is separately prepared, the fourth port is a port to inject a proliferation culture medium, and a fifth port is a port to export an antigen-specific CTL that has been proliferated.

The fourth port is provided in at least the same number as that of the proliferation culture medium. In other words, the number of the fourth ports should be equal to or greater than the number of the proliferation culture media. When the fourth ports with the number greater than the number of the proliferation culture media are provided, a part of such fourth ports can be used as a spare port. The numbers of the first ports, the second ports, the third ports, and the fourth ports are all 1 as a general rule. However, a spare port may be provided in order to cope with an operation error. The spare ports can be provided for each port, or can be provided as a common port for two or more ports (e.g., the third port and the fourth port).

The form or the like of each port is not particularly limited as in the hermetically sealed culture vessel of (1-3). As an example of the constitution of each port, the third port can be a port wherein the injection with a needle is possible (e.g. a port having an injection mouth made of an elastomer such as rubber, etc.) and the other ports can be of a luer lock type. Preferably, from the same reasons as in the hermetically sealed culture vessel of (1-3), at least the fourth port should be of a luer lock type. A part or all of the first to fifth ports may be a branched port. Preferably, at least the fourth port should be a branched port.

As for the preparation kit of the invention, each port provided in all the culture vessels ((1-3), (2-4), and (3-6)) is used for only one time operation. That is to say, a port once used will never be used again in the subsequent operations. Such handling can avoid the occurrence of contamination as much as possible. If a similar handling is applied to the separation vessels of (3-4) and (3-5), contamination during separation operations also can be prevented.

The components contained in the preparation kit of the invention are usually packaged with each component or two or more components together. Preferably, such packages are hermetically sealed.

In one preferable embodiment, two kinds of caps are provided at the first to third ports of the hermetically sealed culture vessel of (1-3), the first to fourth ports of the hermetically sealed culture vessel of (2-4), and the third and fourth ports of the hermetically sealed culture vessel of (3-6), respectively. One cap is attached to a port that is in an unused state. The other cap is attached to a used port. Use of these two kinds of caps makes it possible to easily understand whether each port is unused or has been used. As a result, contaminations can be prevented. In addition, operation errors can be prevented, too. The constitutions (color or form) of these two kinds of caps are not particularly limited as far as both are identifiable. For example, two kinds of caps with different colors are provided. Alternatively, two kinds of caps with different forms are provided.

Examples of the components that can be added to the preparation kit of the invention include a culture medium or vessel used in the preparation of peripheral blood mononuclear cells; a vessel used in the injection of peripheral blood mononuclear cells; a culture medium or vessel used in the preparation of plasma; a vessel used in the injection of the plasma; a separation vessel to separate antigen-specific cytotoxic T lymphocytes being proliferated in the third step; a preservation vessel to cryopreserve the cells being separated using the separation vessel; and a tube used to import/export or dispense the cells. For example, if a culture medium used in the preparation of peripheral blood mononuclear cells and/or a vessel used in the preparation of peripheral blood mononuclear cells is provided, a kit available for the preparation of peripheral blood mononuclear cells will be obtained, thereby increasing convenience or utility value of the kit. Similarly, if a separation vessel to separate antigen-specific cytotoxic T lymphocytes being proliferated in the third step (and a preservation vessel to cryopreserve the cells being separated using the separation vessel concerned) is provided, a kit available for the operations subsequent to the third step will be obtained.

As the culture medium and the vessel used to prepare peripheral mononuclear blood cells, those conventionally used when peripheral mononuclear blood cells are prepared are preferably adopted. Herein, one example of the culture medium can include RPMI 1640 culture medium containing serum albumin and antibiotic, and one example of the vessel can include a disposable centrifugal tube. Meanwhile, as the above separation vessel, those having similar constitution to the separation vessel of (3-1) or (3-2), or commercially available separation bags that are used in the separation of the cells [e.g., separation bag A (Nipro Corp.)] may be adopted. In addition, various bags [e.g. Froze bag (Nipro Corp.)] used for freezing the cells are commercially available, and any one of these can be used as the above-mentioned preservation vessel.

A method for preparing an antigen-specific CTL using the preparation kit of the invention (a second aspect of the invention) will be explained below. The preparation method is roughly made up of three steps: an induction step of antigen-specific CTLs, a preparation step of antigen-presenting cells, and a proliferation step of antigen-specific CTLs. The order of carrying out the first two steps is not limited. In other words, either of them may be performed first. Both of them may be performed in parallel.

### (1) Induction Step of Antigen-specific CTLs

In this step, first, peripheral blood mononuclear cells from the first port and an antigen peptide-containing culture medium (1-1) from the second port are respectively injected into a hermetically sealed culture vessel (1-3). After this operation, the hermetically sealed culture vessel is transferred into an incubator which has been set to the conditions (typically 37°C, 5% CO₂) suitable for the culture of T cells. After a predetermined time (e.g., 1 to 4 days) has passed, the hermetically sealed culture vessel is taken out from the incubator, and an IL-2-containing culture medium (1-2) is injected therein from one of the third ports. Culture is performed in the incubator for a predetermined period (e.g., 2 to 7 days) and then the IL-2-containing culture medium (1-2) is again injected from one (unused one) of the third ports into the hermetically sealed culture vessel. After that, culture in the incubator is continued. When a preparation kit including two IL-2-containing culture media is used, the proliferation step of the antigen-specific CTLs mentioned later is followed after that. When a preparation kit including three or more IL-2-containing culture media is used, an injection operation of the IL-2-containing culture medium and a subsequent culture will be repeated a predetermined number of times (once or twice or more according to the number of the IL-2-containing culture media) before moving to the proliferation step of the antigen-specific CTLs. The interval from the injection operation of the IL-2-containing culture medium to the next injection operation of the IL-2-containing culture medium is, for example, 1 to 6 days. As in an example where the interval between the first injection operation and the second injection operation is 5 days, and the interval between the second injection operation and the third injection operation is 3 days, the interval herein is not necessarily unified. In addition, the preparation of the peripheral blood mononuclear cells may be performed according to a conventional manner. However, in the case of a kit including components for the preparation of the peripheral blood mononuclear cells, the kit will be also utilized at the time of preparing the peripheral blood mononuclear cells.

### (2) Preparation Step of Antigen-Presenting Cells

In this step, first, peripheral blood mononuclear cells from the first port and an anti-CD3 antibody-containing culture medium (2-1) from the second port are respectively injected into a hermetically sealed culture vessel (2-4). After this operation, the hermetically sealed culture vessel is transferred into an incubator which has been set to the conditions (typically 37°C, 5% CO₂) suitable for the culture of T cells. After a predetermined time (e.g., 1 to 4 days), the hermetically sealed culture vessel is taken out from the incubator, and an IL-2-containing culture medium (2-2) is injected therein from one of the third ports. Preferably, the injection operation here uses a culture medium having a lower concentration of IL-2 than the IL-2-containing culture medium used later. Culture is performed in the incubator for a predetermined period (e.g., 2 to 7 days) and then the IL-2-containing culture medium (2-2) is again injected from one (unused one) of the third ports. After that, culture in the incubator is continued. When a preparation kit including two IL-2-containing culture media is used, an injection operation of an antigen peptide-containing culture medium is subsequently performed using the fourth port. After the injection operation, the hermetically sealed culture vessel is allowed to stand for a predetermined time (e.g., 30 minutes to 2 hours). During this time period, the hermetically sealed culture vessel may be stirred at a predetermined interval (e.g., 15-minute interval) while being tilted. The proliferation step of the antigen-specific CTLs mentioned later is conducted after that. When a preparation kit including three or more IL-2-containing culture media is used, an injection operation of the IL-2-containing culture medium and a subsequent culture will be repeated a predetermined number of times (once or twice or more according to the number of the IL-2-containing culture media) before the injection operation of the antigen peptide-containing culture medium. In addition, the interval from the injection operation of the IL-2-containing culture medium to the next injection operation of the IL-2-containing culture medium is, for example, 1 to 6 days. As in an example where the interval between the first injection operation and the second injection operation is 3 days, and the interval between the second injection operation and the third injection operation is 2 days, the interval herein is not necessarily unified.

### (3) Proliferation Step of Antigen-Specific CTL

In this step, the antigen-specific CTLs induced in the induction step of the antigen-specific CTLs and the antigen-presenting cells prepared in the preparation step of the antigen-presenting cells are first collected respectively. The fourth port of the hermetically sealed culture vessel (1-3) is used to collect the antigen-specific CTLs. The fourth port is usually connected to the first port of a first hermetically sealed separation vessel (3-3) by use of a tube, and the antigen-specific CTLs are directly collected in the first hermetically sealed separation vessel. When the hermetically sealed culture vessel (1-3) wherein a tube is attached to the fourth port is used, the antigen-specific CTLs can be collected without separately preparing another tube.

The collection of the antigen-presenting cells is performed by a similar operation. In other words, the fifth port of the hermetically sealed culture vessel (2-4) is usually connected to the first port of a second hermetically sealed separation vessel (3-4) by use of a tube, and the antigen-presenting cells are directly collected in the second hermetically sealed separation vessel.

Then, the antigen-specific CTLs collected in the first hermetically sealed separation vessel are separated. In other words, the first hermetically sealed separation vessel is subjected to a centrifugal treatment to remove unnecessary culture fluid. After that, a culture medium for separating antigen-specific CTLs is injected into the first hermetically sealed separation vessel from the third port to suspend the cells. The fourth port of the first hermetically sealed separation vessel is connected to the first port of a hermetically sealed culture vessel (3-6) by use of a tube so that the cells are transferred to the hermetically sealed culture vessel (3-6) from the first hermetically sealed separation vessel.

On the other hand, the antigen-presenting cells collected in the second hermetically sealed separation vessel are separated in a similar manner to the case of the separation of the antigen-specific CTLs. After that, the fourth port of the second hermetically sealed separation vessel is connected to the second port of the hermetically sealed culture vessel (3-6) by use of a tube so that the cells are transferred from the second hermetically sealed separation vessel to the hermetically sealed culture vessel (3-6). The order of the separation and transfer of the antigen-specific CTLs, and the separation and transfer of the antigen-presenting cells is not important.

Then, after injection of serum or plasma into the hermetically sealed culture vessel (3-6) from the third port, the hermetically sealed culture vessel is transferred into an incubator set to the conditions (37°C, 5% CO₂) suitable for the culture of T cells. However, prior to the transfer of the antigen-specific CTLs and/or the transfer of the antigen-presenting cells, an injection operation of serum or plasma may be performed. After the passage of a predetermined time (e.g., 1 to 4 days), the hermetically sealed culture vessel is taken out from the incubator, and a proliferation culture medium (3-5) is injected from one of the fourth ports. After being cultured in the incubator for a predetermined time (e.g., 2 to 7 days), the proliferation culture medium (3-5) is again injected from one (unused one) of the fourth ports. After that, culture is continued in the incubator. When a preparation kit including two proliferation culture media is used, collection of the cells will be subsequently conducted. When a preparation kit including three or more proliferation culture media is used, an injection operation of the proliferation culture medium and a subsequent culture will be repeated a predetermined number of times (once or twice or more according to the number of the proliferation culture media) before the collection operation. The interval from the injection operation of the proliferation culture medium to the next injection operation of the proliferation culture medium is, for example, 1 to 6 days. As in an example where the interval between the first injection operation and the second injection operation is 3 days, and the interval between the second injection operation and the third injection operation is 2 days, the interval herein is not necessarily unified.

The fifth port of the hermetically sealed culture vessel (3-6) is used for collection operation. In other words, the proliferated antigen-specific CTLs are exported from the fifth port by the above-mentioned operation.

A further aspect (a third aspect) of the invention provides a preparation kit of antigen-specific CTLs using antigen-presenting cells in a freeze-dried state. The preparation kit of the invention includes necessary components for carrying out the induction of antigen-specific CTLs (a first step), the preparation of antigen-presenting cells (a second step), and the proliferation of antigen-specific CTLs (a third step). The components contained in the preparation kit of the invention will be explained with respect to each step. However, explanation of the components in the first step is omitted because it is the same as that of the invention of the first aspect. In addition, with respect to the matters that are not particularly referred to, a corresponding description in the first aspect is quoted.

### (Second Step: Preparation of Antigen-Presenting Cells)

In this step, an antigen-presenting cell in a freeze-dried state is restored to an infiltration state. This processing is also referred to as "reconstruction" in the present specification. For carrying out the step concerned, the preparation kit of the invention includes at least an antigen-presenting cell in a freeze-dried state. The "antigen-presenting cell in a freeze-dried state" is a cell that has acquired an antigen-presenting capacity by the induction with a predetermined antigen and is in a freeze-dried state. The "antigen-presenting cell in a freeze-dried state" can be prepared by the preparation of the antigen presenting cell and the subsequent freeze-drying treatment. For example, the antigen presenting cell can be prepared from dendritic cells or peripheral blood mononuclear cells according to a conventional method. Also, the antigen presenting cells can be prepared from K562 cells, etc. Preferably, K562 cells that have expressed a predetermined HLA (e.g., HLA-A24), CD80, CD83, and CD86 are used as antigen-presenting cells. Freeze-drying treatment of the antigen-presenting cells is preferably performed under the condition such that trehalose is present in a cell suspension. Specific examples of the preparation of the antigen-presenting cells and the freeze-drying treatment are given in the section of Examples described later. An example of the freeze-drying treatment is described in US patent No. 5,059,518. The freeze-drying treatment may be performed according to the treatment method described in the patent. In addition, the contents of the US patent No. 5,059,518 are incorporated herein by reference.

### (Third Step: Proliferation of CTL)

In this step, antigen-specific CTLs are proliferated using the antigen-specific CTLs induced in the first step and the antigen-presenting cells prepared in the second step. For carrying out the step concerned, the preparation kit of the invention includes at least the following components (3-1) to (3-4):
(3-1) a separation medium for antigen-specific cytotoxic T lymphocytes contained in an injection vessel,
(3-2) a first hermetically sealed separation vessel including a first port to import the induced antigen-specific cytotoxic T lymphocytes, a second port to drain waste fluid, a third port to inject a separation medium for the antigen-specific cytotoxic T lymphocytes, and a fourth port to export the antigen-specific cytotoxic T lymphocytes after separation,
(3-3) at least two proliferation culture media contained in an injection vessel, each containing IL-2 or IL-15, or both of them, and
(3-4) a hermetically sealed culture vessel including a first port to import the antigen-specific cytotoxic T lymphocytes after separation, a second port to import the prepared antigen-presenting cells, a third port to inject serum or plasma that is separately prepared, a fourth port(s) to inject the proliferation culture medium wherein the number of the ports is at least the same as the number of the proliferation culture media, and a fifth port to export the proliferated antigen-specific cytotoxic T lymphocytes.

(3-1) is the same as (3-1) of the first aspect, (3-2) is the same as (3-3) of the first aspect, and (3-3) is the same as (3-5) of the first aspect. Therefore, explanations about them are omitted. (3-4) corresponds to (3-6) of the first aspect. The only difference is that the second port is used for importing the antigen-presenting cells which have been prepared. The constitution of the second port concerned is not particularly limited. For example, the second port may be made to have the same constitution as in the second port of (3-6) of the first aspect.

It is possible to add various components (such as a culture medium or vessel used in the preparation of peripheral blood mononuclear cells; a vessel used in the injection of peripheral blood mononuclear cells; a culture medium or vessel used in the preparation of plasma; a vessel used in the injection of plasma; a culture medium or vessel used in the reconstruction of antigen-presenting cells; a separation vessel for separating antigen-specific cytotoxic T lymphocytes proliferated in the third step; a preservation vessel to cryopreserve the cells that are separated using the separation vessel; and a tube used to import/export or dispense the cells) in the same manner as in the preparation kit of the first aspect.

The preparation method for antigen-specific CTLs (the fourth aspect of the invention) with use of the preparation kit of the invention is the same as the case using the preparation kit of the first aspect of the invention, except for the second step. The second step of the present aspect reconstructs antigen-presenting cells in a freeze-dried state. The reconstruction method typically comprises infiltrating the cells and washing (suspending) the cells. Specifically, a sufficient amount of solvents (e.g. deionized water, distilled water, saline, etc.) is first added to the antigen-presenting cells in a freeze-dried state, and allowed to stand for a while (infiltration step). After addition of a culture medium, the mixture is subjected to a centrifugal treatment (washing step). The supernatant was removed by aspiration, and a culture medium is added again to suspend the cells (suspending step).

Hereinafter, the present invention will be explained in more details by using Examples.

### Example 1

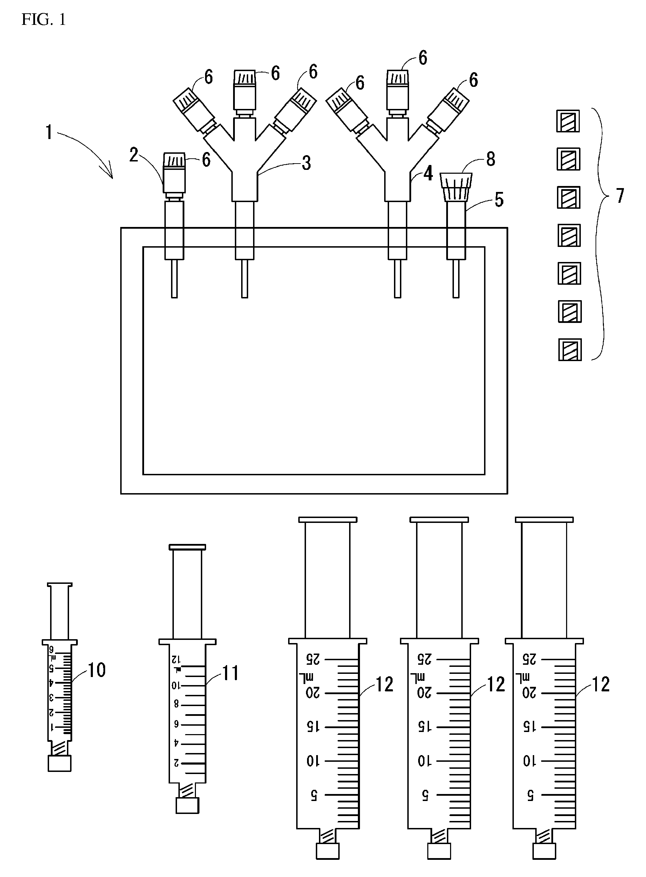

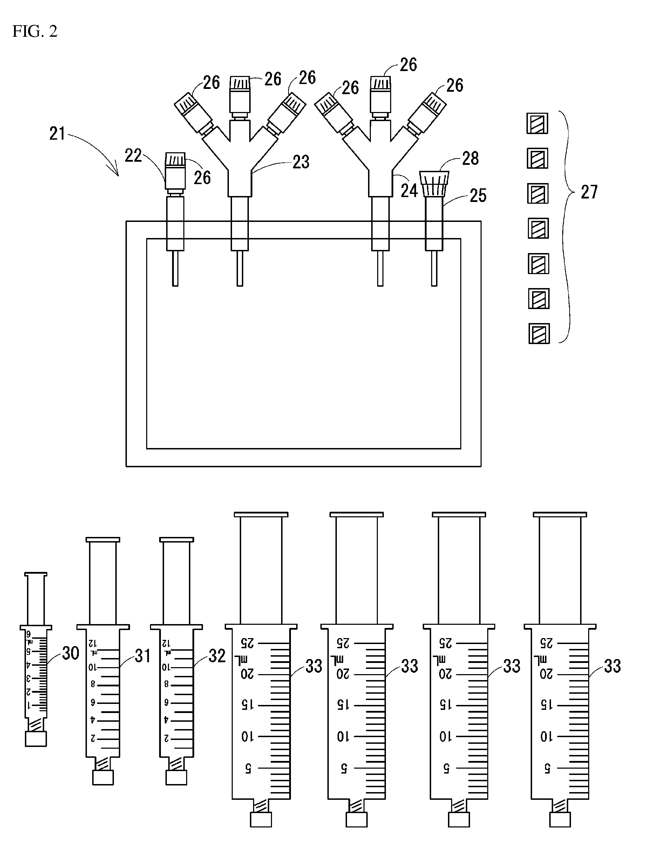

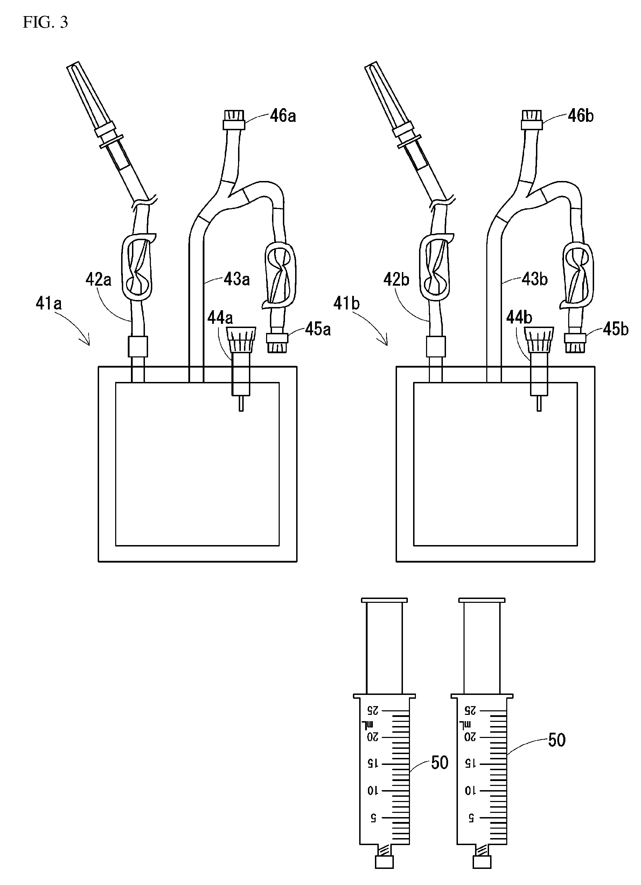

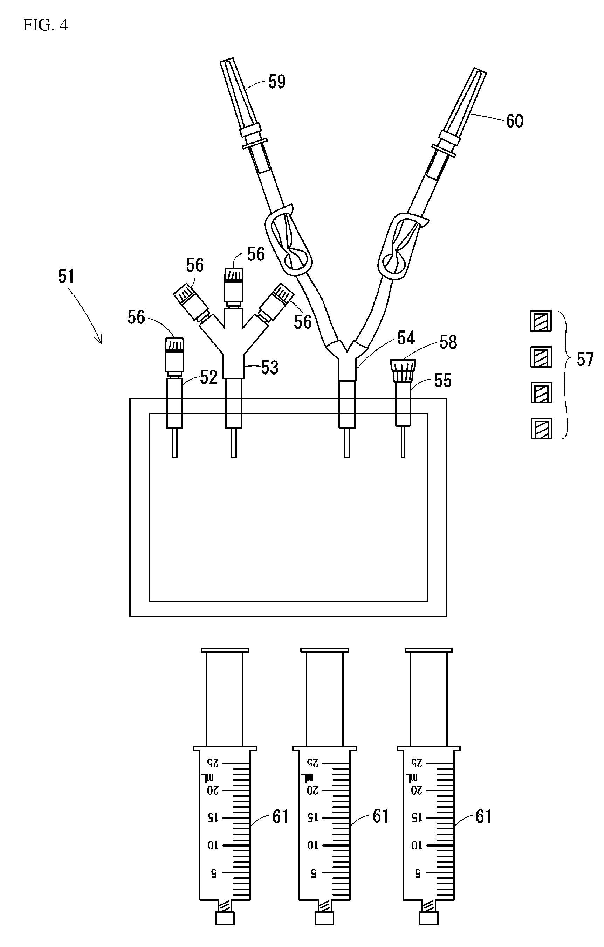

The components (constituent parts) of this Example (preparation kit of antigen-specific CTLs) of the invention are shown in Figs. 1 to 4. Fig. 1 shows one set of the components used in the induction step of antigen-specific CTLs (components for the induction of antigen-specific CTLs), Fig. 2 shows one set of the components used in the preparation step of antigen-presenting cells (components for the preparation of antigen-presenting cells), and Figs. 3 and 4 show one set of the components used in the proliferation step of antigen-specific CTLs (components for the proliferation of antigen-specific CTLs).

The breakdowns of the components for the induction of antigen-specific CTLs (Fig. 1) are as follows.
Culture bag 1....One
Antigen peptide-containing culture medium 10 (Hepes buffer RPMI 1640 containing 0.01% human serum albumin and 2 µg CMVpp65 (antigen peptide)) of 5 mL contained in a luer lock syringe....One
IL-2-containing culture medium 11 (Hepes buffer RPMI 1640 containing 0.01 % human serum albumin and 100 IU/mL IL-2) of 10 mL contained in a luer lock syringe....One
IL-2-containing culture medium 12 (ALyS505N (Cell Science & Technology Institute, Inc.) containing 100 IU/mL IL-2) of 20 mL contained in a luer lock syringe....Three

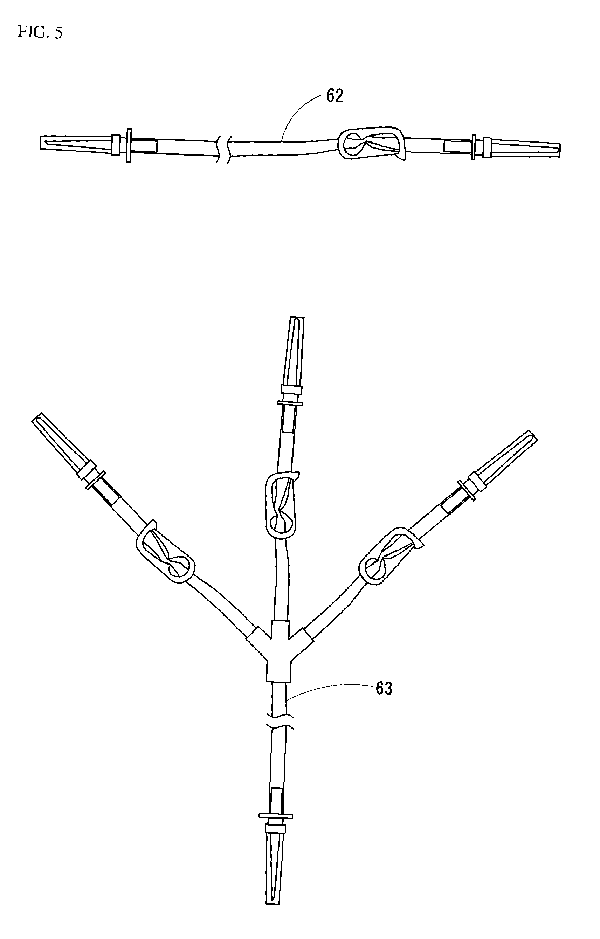

The culture bag 1 comprises a luer lock type port 2, luer lock type 3-branched ports (3, 4), and a port 5 for a plastic needle (see Fig. 5). The port 2 is used for the injection of peripheral blood mononuclear cells that are separately prepared. In addition, the 3-branched ports (3, 4) are combined use ports that are used to inject an antigen peptide-containing culture medium 10 and to inject IL-2-containing culture media 11, 12. The port 5 is used to export the induced antigen-specific CTLs. All the ports except for the port 5 are of a luer lock type. A cap 6 is attached to the port 2 and 3-branched ports (3, 4) when in no use. The reference numeral 7 shows caps to be attached to the ports (2, 3, 4) after use, and these caps differ in the color from the cap 6 that is attached when in no use. In this example, the cap 6 was made blue and the cap 7 was made colorless transparent. A cap 8 is also attached to the port 5 when in no use. The material of the culture bag 1 was made of polyethylene.

The breakdowns of the components for the preparation of antigen-presenting cells (Fig. 2) are as follows.
Culture bag 21.... One
Anti-CD3 antibody-containing culture medium 30 (Hepes buffer RPMI 1640 containing 0.01% human serum albumin and 2 µg/mL OKT-3 (anti-CD3 antibody) of 5 mL contained in a luer lock syringe....One
IL-2-containing culture medium 31 (Hepes buffer RPMI 1640 containing 100 IU/mL IL-2) of 10 mL contained in a luer lock syringe....One
Antigen peptide-containing culture medium 32 (Hepes buffer RPMI 1640 containing 0.01% human serum albumin and 2 µg/mL CMVpp65 (antigen peptide) of 10 mL contained in a luer lock syringe....One
IL-2-containing culture medium 33 (ALyS505N (Cell Science & Technology Institute, Inc.) containing 1000 IU/mL IL-2) of 20 mL....Four

The culture bag 21 comprises a luer lock type port 22, luer lock type 3-branched ports (23, 24), and a port 25 for a plastic needle. The port 22 is used for the injection of peripheral blood mononuclear cells that are separately prepared. In addition, the 3-branched ports (23, 24) are combined use ports that are used to inject an anti-CD3 antibody-containing culture medium 30, an IL-2-containing culture media (31, 33), and an antigen peptide-containing culture medium 32. The port 25 is used to export the prepared antigen-presenting cells. All the ports except for the port 25 are of a luer lock type. Cap 26 is attached to the port 22 and 3-branched ports (23, 24) when in no use. The reference numeral 7 shows caps to be attached to the ports (22, 23, 24) after use, and these caps differ in the color from cap 26 being attached when in no use. In this example, cap 26 was made blue and cap 27 was made colorless transparent. Cap 28 is also attached to the port 25 when in no use. The material of the culture bag 21 was made of polyethylene.

The breakdowns of the components (Figs. 3, 4) for the proliferation of antigen-specific CTLs are as follows.
Separation bags (41a, 41b)....Two
IL-2-containing culture medium 50 (ALyS505N (Cell Science & Technology Institute, Inc.) containing 100 IU/mL IL-2) of 20 mL contained in a luer lock syringe....Two
Culture bag 51.... tone
Proliferation culture medium 61 (ALyS505N (Cell Science & Technology Institute, Inc.) containing 1000 IU/mL IL-2) of 20 mL contained in a luer lock syringe....Three

The separation bags (41a, 41b) comprise ports (42a, 42b) wherein a plastic needle is connected through a tube, luer lock type 2-branched ports (43a, 43b), and ports (44a, 44b) for plastic needles. One (41a) of the separation bags is used in the separation of the induced antigen-specific CTLs. In the separation bag concerned, the port 42a is used for importing the induced antigen-specific CTLs. The 2-branched port 43a is used for draining waste fluid as well as for injecting the IL-2 containing culture medium 50. The port 44a is used in exporting the cells after treatment. On the other hand, the separation bag 41b is used in the separation of antigen-presenting cells that have been prepared. In the separation bag concerned, the port 42b is used for importing the antigen-presenting cells. The 2-branched port 43b is used for draining waste fluid as well as for injecting the IL-2 containing culture medium 50. The port 44b is used in exporting the cells after treatment. The materials of the separation bags (41a, 41b) were made of polyethylene.

The culture bag 51 comprises a luer lock type port 52, a luer lock type 3-branched port 53, a 2-branched port 54 wherein a plastic needle is connected through a tube, and a port 55 for a plastic needle. The port 52 is used for the injection of plasma that is separately prepared. In addition, the 3-branched port 53 is used to inject a proliferation culture medium 61. The port 54 is used for importing the separated antigen-specific CTLs and antigen-presenting cells. The port 55 is used for exporting the proliferated antigen-specific CTLs. Cap 56 is attached to the port 52 and the 3-branched port 53 when in no use. The reference numeral 57 shows caps to be attached to the ports (52, 53) after use, and these caps differ in the color from cap 56 being attached when in no use. In this example, cap 56 was made blue and cap 57 was made colorless transparent. Cap 58 is also attached to the port 55 when in no use. The material of the culture bag 51 was made of polyethylene.

HLA-A24-restricted CMV pp65 antigen-specific CTL was prepared by the following step using the preparation kit with the above constitution. Details of each step will be explained with reference to Figs. 6 to 8.

### (1) CTL Induction Step (Fig. 6)

### (a) Separation of Peripheral Blood Mononuclear Cell (PBMC)

A blood sample of 50 mL was collected from an HLA-A24 positive healthy volunteer with a syringe to which a small amount of heparin had been added, and PBMCs were separated by a Ficoll density gradient centrifugation technique (4×10⁷).

### (b) Collection of Plasma

Plasma was collected at the time of separating PBMCs, treated under heating at 56°C for 20 minutes, and centrifuged at 2500 rpm for 10 minutes.

### (c) Suspension of PBMC in RPMI 1640 Culture Medium

As shown in the Figure, after washing PBMCs with a culture medium in a bag (Hepes buffer RPMI 1640 medium containing 0.01 % human serum albumin and 10 µ/mL gentacin), 4 mL of the bag medium was collected and suspended with PBMCs. Further, autologous plasma of 1mL was added to the suspension (PBMC density: 6×10⁶/mL) .

### (d) Induction of CTL

PBMCs suspended in RPMI 1640 culture medium were injected into a culture bag 1 from a port 2 with a luer lock syringe (see Fig. 1). Then, an antigen peptide-containing culture medium 10 was injected into the culture bag 1 from any one of the ports of a 3-branched port 3 (or a 3-branched port 4), and then the culture bag 1 was transferred into a CO₂-incubator of 37°C and 5% CO₂ (start of culture). In addition, the cap 7 in place of the cap 6 is attached to the port once used, and such a port is not used again.

The culture bag 1 was taken out from the incubator two days later. Then, an IL-2-containing culture medium 11 was injected into the culture bag 1 from any one of the ports (excluding the port already used) of a 3-branched port 3 (or a 3-branched port 4). Culture was performed in a CO₂-incubator for 5 days, and then an IL-2-containing culture medium 12 was injected into the culture bag 1 from any one of the ports (excluding the port already used) of a 3-branched port 3 (or a 3-branched port 4). Afterwards, culture (3 days), injection of the IL-2-containing culture medium 12, culture (2 days), injection of the IL-2-containing culture medium 12, and culture (2 days) were performed in this order. By the above-mentioned operations, antigen-specific CTLs were induced in a short time from the start of culture to day 14 (provided that the time required for the preparation of PBMC and plasma is excluded).

A culture medium in a bag for use in the preparation of PBMCs may be attached to the preparation kit. Similarly, a tube used for Ficoll separation, a tube used for washing PBMCs, and a tube used for resuspending PBMCs may be attached to the preparation kit.

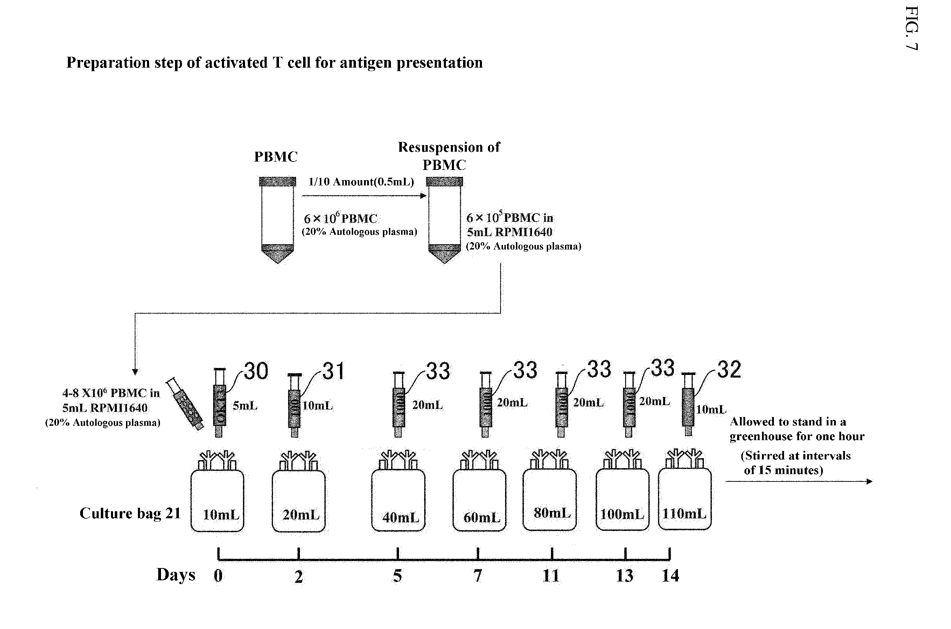

### (2) Preparation Step of Antigen-Presenting Cells (Fig. 7)

### (a) Suspension of PBMC into RPMI 1640 Culture Medium

A culture medium (3.6 mL) in a bag and autologous plasma (0.9 mL) were added to PBMCs (0.5 mL) which had been prepared in (1)(a)(PBMC density: 6x 10⁵/mL).

### (b) Preparation of Antigen-Presenting Cells

PBMCs suspended in RPMI 1640 culture medium were injected into a culture bag 21 from a port 22 with a luer lock syringe (see Fig. 2). Then, an anti-CD3 antibody-containing culture medium 30 was injected into the culture bag 21 from any one of the ports of a 3-branched port 23 (or a 3-branched port 24), and the culture bag 21 was then transferred into a CO₂-incubator of 37°C and 5% CO₂ (start of culture). A cap 27 in place of a cap 26 is attached to the port once used, and such a port is not used again.

The culture bag 21 was taken out two days later. Then, an IL-2-containing culture medium 31 was injected into the culture bag 21 from any one of the ports (excluding the port already used) of a 3-branched port 23 (or a 3-branched port 24). Culture was performed in a CO₂-incubator for 3 days, and then an IL-2-containing culture medium 33 was injected into the culture bag 21 from any one of the ports (excluding the port already used) of a 3-branched port 23 (or a 3-branched port 24). Afterwards, culture (2 days), injection of the IL-2-containing culture medium 33, culture (4 days), injection of the IL-2-containing culture medium 33, culture (2 days), injection of the IL-2-containing culture medium 33, and culture (1 day) were performed in this order. By the above-mentioned operations, antigen-presenting cells were prepared in a short time from the start of culture to day 14. In this Example, the preparation step (2) of the antigen-presenting cells was performed in parallel with the induction step (1) of CTLs. Therefore, both of the antigen-specific CTLs and the antigen-presenting cells are obtained in 14 days in total (excluding the time required for the preparation of PBMCs and plasma).

### (c) Peptide Pulse to Antigen-Presenting Cells

An antigen peptide-containing culture medium 32 was injected into a culture bag 21 from any one of the ports of a 3-branched port 23 (or a 3-branched port 24) (excluding the port already used). After that, incubation was performed at room temperature for one hour.

### (3) Proliferation Step of Antigen-Specific CTL (Fig. 8)

### (a) Separation of Antigen-Presenting Cell and Antigen- Specific CTL

A plastic needle connected to a port 42b of a separation bag 41b was inserted into a port 25 of the culture bag 21, and the contents of the culture bag 21 were transferred to the separation bag 41b (see Fig. 3). After closing a clamp of the port 42b (the tube may be welded with a sealer), the separation bag 41b was subjected to centrifuge. The bag was pressed while the clamp of a 2-branched port 43b was being kept open, the supernatant was discarded from a drainage mouth 45b of the 2-branched port, and an IL-2-containing culture medium 50 was injected into the separation bag 41b from an injection mouth 46b of the 2-branched port. Meanwhile, a plastic needle connected to a port 42a of a separation bag 41a was inserted into a port 5 of a culture bag 1 after the above step (1) so that the contents of the culture bag 1 was transferred to the separation bag 41a, and then the same operations as above (centrifuge, drainage, injection of IL-2-containing culture medium 50) were performed.

### (b) Mixing and Culture of Antigen-Presenting Cells and Antigen-specific CTLs

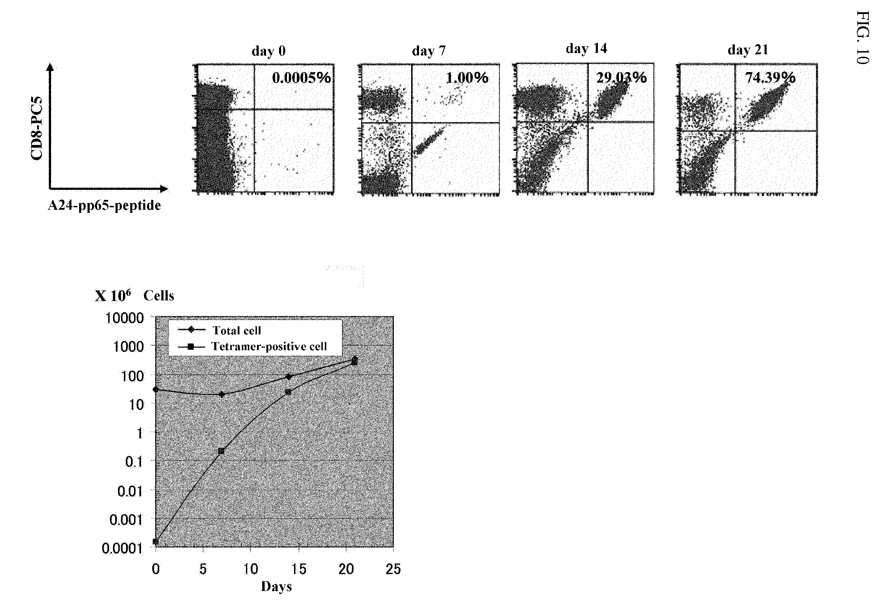

One needle 59 of the plastic needles connected to a port 54 of a culture bag 51 was inserted into a port 44a of a separation bag 41a, and the contents of the culture bag 41a were transferred to the culture bag 51 (see Figs. 3 and 4). Similarly, a plastic needle 60 was inserted into a port 44b of a separation bag 41b, and the contents of the culture bag 41b were transferred to the separation bag 51. Subsequently, autologous plasma that had been prepared in the step (1)(b) was injected into the culture bag 51 from a port 52 with a luer lock syringe, and the culture bag 51 was transferred into a CO₂-incubator of 37°C and 5% CO₂ (start of culture). After one day had passed, the culture bag 51 was taken out. A proliferation culture medium 61 was injected into the culture bag 51 from any port of 3-branched ports 53. A cap 57 in place of a cap 56 is attached to the port once used, and such a port is not used again. Culture was performed in a CO₂-incubator for 3 days, and then a proliferation culture medium 61 was injected into the culture bag 51 from any one of the ports (excluding the port already used) of the 3-branched port 53. Afterwards, culture (2 days), injection of the proliferation culture medium 61, and culture (1 day) were performed in this order. By the above-mentioned operations, antigen-specific CTLs were obtained in a short time from the start of culture in the steps (1) and (2) to day 21. The obtained cells were subjected to flow cytometry analysis. The results revealed that HLA-A24-restricted CMV pp65 antigen-specific CTLs with a purity of 74.39% was obtained in 3.26×10⁸ (Fig.10). In other words, in spite of a short time of 21 days (excluding the time required for the preparation of PBMC and plasma), antigen-specific CTLs were successfully prepared in a high purity.

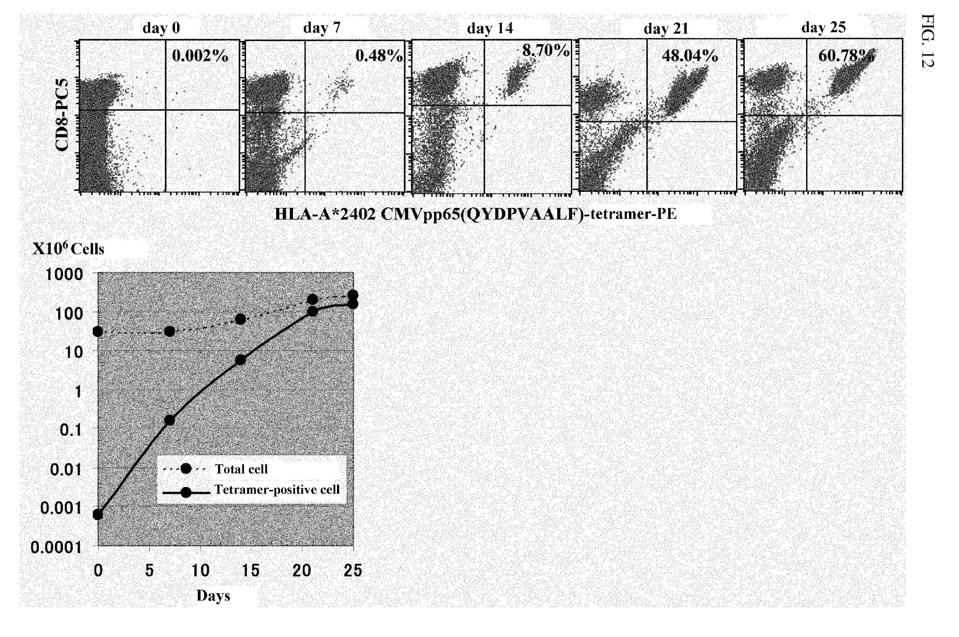

A preparation of antigen-specific CTLs by a similar technique from different donors (three donors) was tried (Figs. 11 to 14). From one donor (HLA-A24 positive), preparation was performed two times under the same conditions (Figs. 11 and 12). Only an example of Fig. 14 is different in an HLA type of the donor (HLA-A2 positive). As shown in Figs. 11 to 14, a high purity of antigen-specific CTL could be prepared with a good reproduction. In addition, the prepared antigen-specific CTL showed a high cytotoxic activity (lower right part of Fig. 11, lower right part of Fig. 13, and lower right part of Fig. 14).

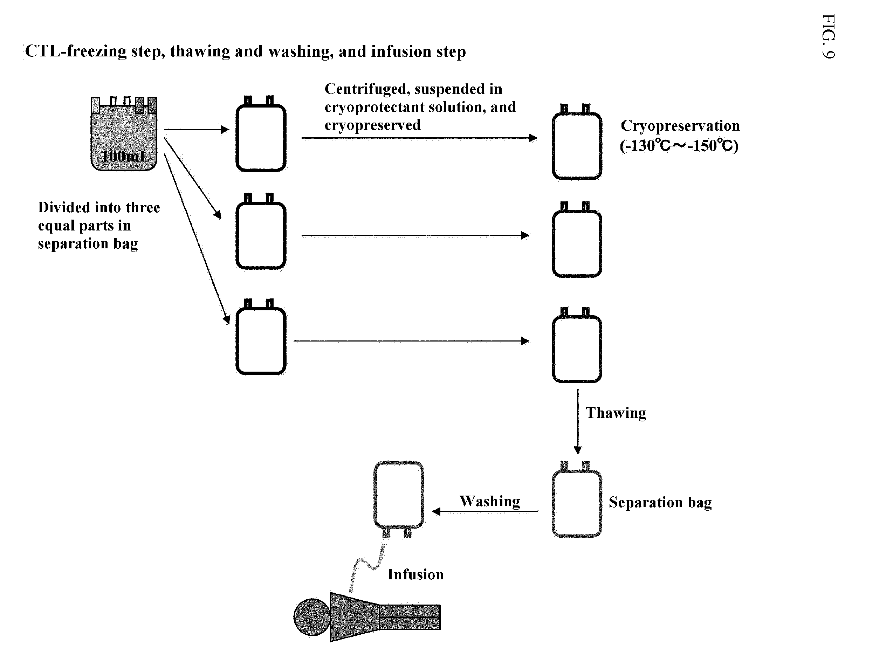

An example of the operation after the antigen-specific CTLs has been prepared is shown in Fig. 9. The contents (i.e., antigen-specific CTLs) of the preparation bag were dispensed into a separation bag using a port 55 of a culture bag 51 (see Fig. 4). This operation can be performed using a 3-branched tube 63 shown in Fig. 5. Then, the supernatant (culture fluid) after centrifuge was discarded, and a cryoprotectant solution (CP-1 (Kyokuto Pharmaceutical Industrial Co., Ltd.) supplemented with 8% human serum albumin) is injected to suspend the cells. The cell suspension is then exported to a cryopreservation bag, and cryopreserved therein (e.g., -130°C to -150°C). The cryopreserved cell suspension is thawed, washed with a separation bag (e.g., a bag having a constitution according to a separation bag 41), and the cells are infused to a patient.

### Example 2

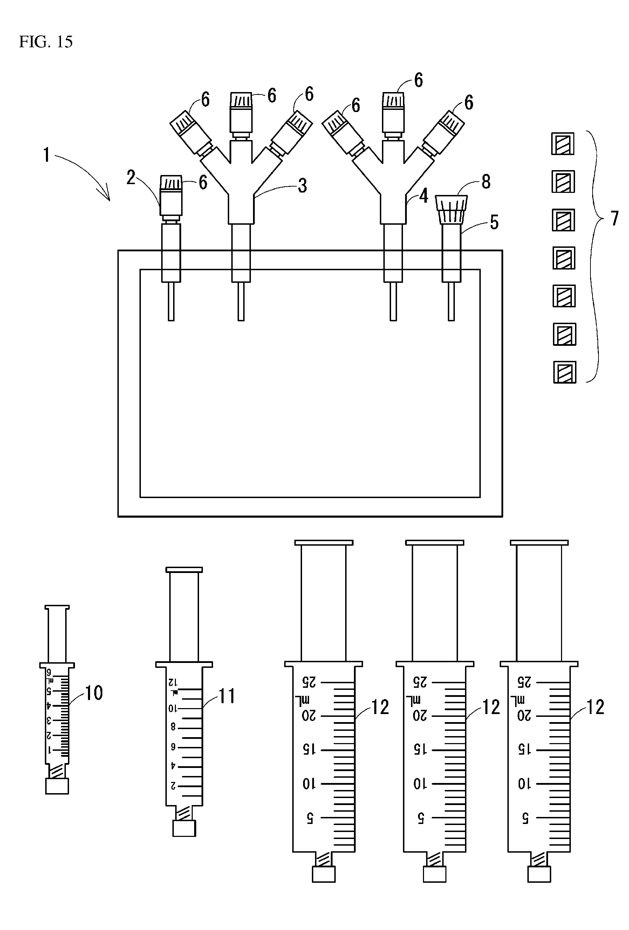

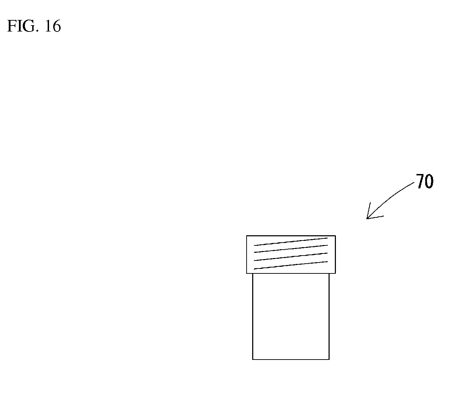

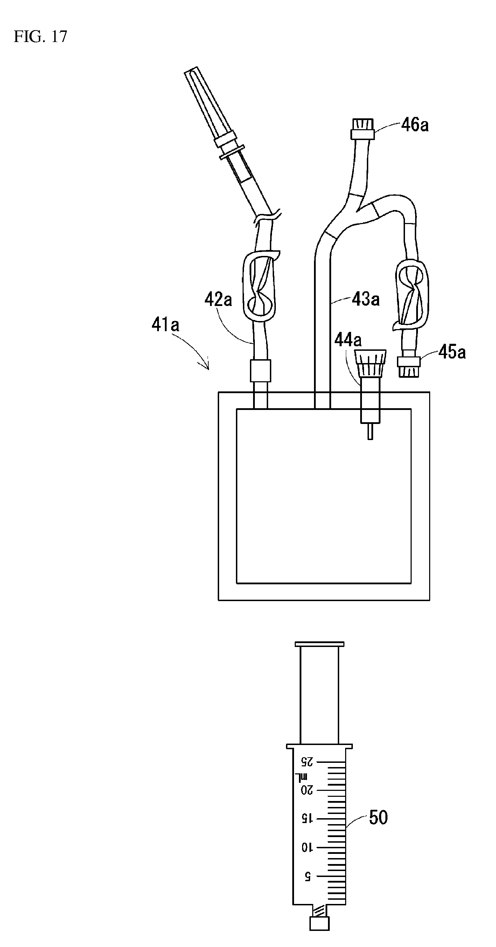

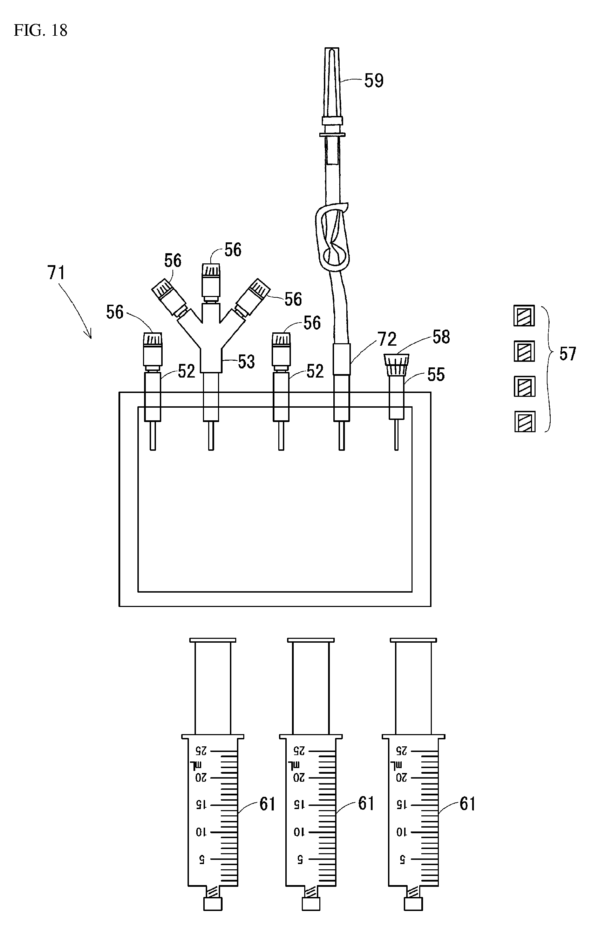

The components (constituent parts) of the Example (a preparation kit of antigen-specific CTLs containing freeze-dried antigen-presenting cells) of the invention are shown in Figs. 15 to 18. Fig. 15 shows one set of the components used in the induction step of antigen-specific CTLs (components for the induction of antigen-specific CTLs), Fig. 16 shows one set of the components used in the preparation step of antigen-presenting cells (components for the preparation of antigen-presenting cells), and Figs. 17 and 18 show one set of the components used in the proliferation step of antigen-specific CTLs (components for the proliferation of antigen-specific CTLs). Incidentally, the same component as in Example 1 bears the same reference numeral.

The breakdowns of the components for the induction of antigen-specific CTLs (Fig. 15) are as follows. The explanation of the constitution of each component is omitted because it is the same as in Example 1.
Culture bag 1....One
Antigen peptide-containing culture medium 10 (Hepes buffer RPMI 1640 containing 0.01% human serum albumin and 2 µg/mL CMVpp65 (antigen peptide)) of 5 mL contained in a luer lock syringe....One
IL-2-containing culture medium 11 (Hepes buffer RPMI 1640 containing 0.01 % human serum albumin and 100 IU/mL IL-2) of 10 mL contained in a luer lock syringe....One
IL-2-containing culture medium 12 (ALyS505N (Cell Science & Technology Institute, Inc.) containing 100 IU/mL IL-2) of 20 mL contained in a luer lock syringe....Three

The breakdowns of the component for the preparation of antigen-presenting cells (Fig. 16) are as follows.
Antigen-presenting cells 70 in a freeze-dried state contained in a vessel (vial in this case)....One

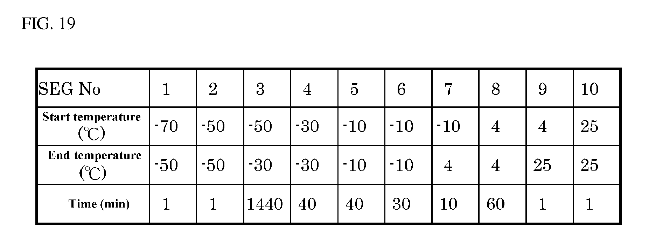

An antigen-presenting cell having an antigen-presenting capacity depending on purposes is used. An example of the preparation of the freeze-dried antigen-presenting cells is shown below. It is common to use a dendritic cell as an antigen-presenting cell, but an activated T cell is herein prepared as an antigen-presenting cell.
(1) Isolation of Peripheral Blood Mononuclear Cells
   A peripheral blood sample was collected from an HLA-A24 positive healthy volunteer with a syringe to which a small amount of heparin had been added. Then, the peripheral blood mononuclear cells were isolated by a Ficoll density gradient centrifugation technique.
(2) Start of Culture
   After washing three times with 10 mL RPMI 1640, the peripheral blood mononuclear cells were suspended again in 10 mL RPMI 1640 (containing 10% autologous plasma and 1 µg/mL OKT-3) so that they resulted in a density of 4×10⁵/mL, and injected into a culture bag. Culture was started in a CO₂-incubator at 37°C.
(3) Addition of IL-2
   Two days later, 10mL RPMI 1640 (containing 1 µg/mL of 100 IU/mL IL-2) was injected, and culture was performed in a CO₂-incubator (37°C) for 3 days.
(4) Addition of ALyS505N (Containing IL-2 of 1,000 IU/mL) Culture Medium)
   After addition of 20 mL of an ALyS505N (containing IL-2 of 1,000 IU/mL) culture medium, culture was performed in a CO₂-incubator for 9 days while the ALyS505N (containing IL-2 of 1,000 IU/mL) culture medium (20 mL) was added every four days (total culture days: 14 days).
(5) Measurement of Cell Count
   As a result of measuring the cell count, it was found that 1.53×10⁸ cells were obtained.
(6) Detection of CD80 and CD86
   As a result of examination on the expression of cell surface antigens, it was found that 80% of the cells were positive to CD80, and 85% of the cells were positive to CD86 (The results are not shown in the Figure).
(7) Epitope Peptide Pulse
   After the above prepared antigen-presenting cells were resuspended in a PRMI 1640 to a cell density of 2x 10⁶/mL, an HLA-A24 restricted-CMV pp65-antigen epitope peptide (QYDPVLAALF) was added thereto to a density of 10 µg/mL. Incubation was performed at room temperature for 30 minutes and then the cells were washed three times with 10 mL RPMI 1640.
(8) Freeze-Drying
   The peptide-pulsed cells were resuspended in ISOTON (registered trade mark) III containing 10% trehalose to a cell density of 2×10⁶/mL. The suspension was dispensed (1 mL each) into a vial and then freeze-dried under the conditions as shown in Fig. 19.

The breakdowns of the components for the proliferation of antigen-specific CTLs (Figs. 17 and 18) are as follows. The explanation of the constitution of each component is omitted because it is the same as in Example 1.
Separation bag 41a....One
IL-2-containing culture medium 50 (ALyS505N (Cell Science & Technology Institute, Inc.) containing 100 IU/mL IL-2) of 20 mL contained in a luer lock syringe....One
Culture bag 71.... One
Proliferation culture medium 61 (ALyS505N (Cell Science & Technology Institute, Inc.) containing 1000 IU/mL IL-2) of 20 mL contained in a luer lock syringe....Three

Two luer lock type ports 52 are provided in a culture bag 71. One of the ports is used to inject the plasma that is separately prepared and the other is used to inject the antigen-presenting cells. Meanwhile, a port 72 to which a plastic needle is connected through a tube is a port to import the separated antigen-specific CTLs.

When the preparation kit for antigen-specific CTLs of this Example is used, antigen-specific CTLs are induced using the components for inducing the antigen-specific CTLs and antigen-presenting cells are reconstructed using the components for preparing the antigen-presenting cells. The thus obtained two kinds of cells are co-cultured in the culture bag 71 of the components for the proliferation of the antigen-specific CTLs, thereby to obtain the target antigen-specific CTLs. Further, preparation (reconstruction) of the antigen-presenting cells is carried out as follows.
(1) Addition of Deionized Water
   Deionized water of 1 mL is added to a vial 70 and the vial is allowed to stand at room temperature for 5 minutes.
(2) Washing
   The cells are transferred to a 15 mL-centrifugal tube containing RPMI 1640 (containing 0.05% HSA) of 1 mL, and centrifuged at 1200 rpm and room temperature for 10 minutes. After removal of the supernatant by aspiration, RPMI 1640 of 1 mL (containing 5% autologous plasma) is added to suspend the cells.

### INDUSTRIAL APPLICABILITY

As a matter of course, the time required for preparing the desired antigen-specific CTLs depends on therapeutic purposes and conditions of patients, but it becomes possible to prepare a therapeutically necessary amount of antigen-specific CTLs from isolated peripheral blood mononuclear cells in about 20 days to 2 months.

The present invention is not limited at all by the explanations of the embodiments and Examples of the above-mentioned invention. Various modified embodiments are also encompassed in the present invention to the extent that a person skilled in the art can readily conceive, without departing from the description of the claims.
Contents of the articles, unexamined patent publications and granted patent publications disclosed in the present specification are incorporated herein by reference in their entirety.

### EXPLANATION OF REFERENCES NUMERAL

1: Culture bag
2: Luer lock port
3 and 4: 3-branched luer lock ports
5: Export port
6, 7 and 8: Caps
10: Antigen peptide-containing culture medium (5 mL)
11: IL-2-containing culture medium (10 mL)
12: IL-2-containing culture medium (20 mL)
21: Culture bag
22: Luer lock port
23 and 24: 3-branched luer lock ports
25: Export port
26, 27 and 28: Caps
30: Anti-CD3 antibody-containing culture medium (5 mL)
31: IL-2-containing culture medium (10 mL)
32: Antigen peptide-containing culture medium (10 mL)
33: IL-2-containing culture medium (20 mL)
41a and 41b: Separation bags
42a and 42b: Import ports
43a and 43b: 2-branched luer lock ports
44a and 44b: Export ports
50: IL-2-containing culture medium
51: Culture bag
61: Proliferation culture medium
62: Import/Export tube
63: 3-branched tube for dispensing
70: Vial (containing antigen-presenting cells in a freeze-dried state)

## Claims

1. A preparation kit used for a method for preparing an antigen-specific cytotoxic T lymphocyte, the method comprising: a first step wherein an antigen-specific cytotoxic T lymphocyte is induced; a second step wherein an activated T cell for antigen presentation is prepared; and a third step wherein the antigen-specific cytotoxic T lymphocyte is proliferated using the antigen-specific cytotoxic T lymphocyte induced in the first step and the activated T cell for antigen presentation prepared in the second step;
the components in the first step comprising:
(1-1) an antigen peptide-containing culture medium contained in an injection vessel,
(1-2) at least two culture media, each containing IL-2 contained in an injection vessel, and
(1-3) a hermetically sealed culture vessel comprising a first port to inject a peripheral blood mononuclear cell that is separately prepared, a second port to inject the antigen peptide-containing culture medium, a third port(s) to inject the antigen peptide-containing culture medium wherein the number of the ports is at least the same as the number of the antigen peptide-containing culture media, and a fourth port to export the induced antigen-specific cytotoxic T lymphocyte,
the components in the second step comprising:
(2-1) an anti-CD3 antibody-containing culture medium contained in an injection vessel,
(2-2) at least two culture media, each containing IL-2 contained in an injection vessel,
(2-3) an antigen peptide-containing culture medium contained in an injection vessel, and
(2-4) a hermetically sealed culture vessel comprising a first port to inject a peripheral blood mononuclear cell that is separately prepared, a second port to inject the anti-CD3 antibody-containing culture medium, a third port(s) to inject the IL-2-containing culture medium wherein the number of the ports is at least the same as the number of the IL-2-containing culture media, a fourth port to inject the antigen peptide-containing culture medium, and a fifth port to export the activated T cell for antigen presentation that has been prepared, and
the components in the third step comprising:
(3-1) a separation medium for antigen-specific cytotoxic T lymphocytes contained in an injection vessel,
(3-2) a separation medium for activated T cells for antigen presentation contained in an injection vessel,
(3-3) a first hermetically sealed separation vessel comprising a first port to import the induced antigen-specific cytotoxic T lymphocyte, a second port to drain waste fluid, a third port to inject the separation medium for antigen-specific cytotoxic T lymphocytes, and a fourth port to export the antigen-specific cytotoxic T lymphocyte after separation,
(3-4) a second hermetically sealed separation vessel comprising a first port to import the activated T cell for antigen presentation that has been prepared, a second port to drain waste fluid, a third port to inject a separation medium for the activated T cells for antigen presentation, and a fourth port to export the activated T cell for antigen presentation,
(3-5) at least two proliferation culture media, each containing IL-2 or IL-15, or both of them, contained in an injection vessel, and
(3-6) a hermetically sealed culture vessel comprising a first port to import an antigen-specific cytotoxic T lymphocyte after separation, a second port to import a cell for antigen presentation after separation, a third port to inject serum or plasma that is separately prepared, a fourth port(s) to inject the proliferation culture medium wherein the number of the ports is at least the same as the number of the proliferation culture media, and a fifth port to export the proliferated antigen-specific cytotoxic T lymphocyte.

2. The preparation kit according to claim 1, wherein the number of the IL-2-containing culture media in the above (1-2) is 3 to 5.

3. The preparation kit according to claim 1 or 2, wherein the number of the IL-2-containing culture media in the above (2-2) is 3 to 6.

4. The preparation kit according to any one of claims 1 to 3, wherein the separation medium for antigen-specific cytotoxic T lymphocyte in the above (3-1) and the separation medium for activated T cells for antigen presentation in the above (3-2) are each an IL-2-containing culture medium.

5. The preparation kit according to any one of claims 1 to 4, wherein two kinds of caps are provided at the first to third ports of the hermetically sealed culture vessel in the above (1-3), the first to fourth ports of the hermetically sealed culture vessel in the above (2-4), and the third and fourth ports of the hermetically sealed culture vessel in the above (3-6), respectively, the two kinds of caps each being a cap attached when not in use and a cap that is distinguishable from the cap and attached after use.

6. The preparation kit according to any one of claims 1 to 5, wherein the third port of the hermetically sealed culture vessel in the above (1-3), the third port of the hermetically sealed culture vessel in the above (2-4), and the fourth port of the hermetically sealed culture vessel in the above (3-6) are each a branched port.

7. The preparation kit according to any one of claims 1 to 6, wherein each of the hermetically sealed culture vessel in the above (1-3), the hermetically sealed culture vessel in the above (2-4), and the hermetically sealed culture vessel in the above (3-6) includes a spare port.

8. The preparation kit according to any one of claims 1 to 7, wherein a culture medium for peripheral blood mononuclear cells contained in a vessel is further comprised.

9. The preparation kit according to claim 8, wherein a vessel for preparing peripheral blood mononuclear cells is further comprised.

10. The preparation kit according to any one of claims 1 to 9, wherein a separation vessel for separating the antigen-specific cytotoxic T lymphocytes that have been proliferated in the third step is further comprised.

11. The preparation kit according to claim 10, wherein a preservation vessel to cryopreserve the cells that have been separated using the separation vessel is further comprised.

12. A preparation kit used for a method for preparing an antigen-specific cytotoxic T lymphocyte, the method comprising: a first step wherein an antigen-specific cytotoxic T lymphocyte is induced; a second step wherein an antigen-presenting cell is prepared; and a third step wherein the antigen-specific cytotoxic T lymphocytes are proliferated using the antigen-specific cytotoxic T lymphocyte induced in the first step and the antigen-presenting cell prepared in the second step;
the components in the first step comprising:
(1-1) an antigen peptide-containing culture medium contained in an injection vessel,
(1-2) at least two culture media, each containing IL-2, contained in an injection vessel, and
(1-3) a hermetically sealed culture vessel comprising a first port to inject a peripheral blood mononuclear cell that is separately prepared, a second port to inject the antigen peptide-containing culture medium, a third port(s) to inject the antigen peptide-containing culture medium wherein the number of the ports is at least the same as the number of the antigen peptide-containing culture media, and a fourth port to export the induced antigen-specific cytotoxic T lymphocyte,
the components in the second step comprising:
an antigen-presenting cell in a freeze-dried state contained in a vessel and
the components in the third step comprising:
(3-1) a separation medium for antigen-specific cytotoxic T lymphocytes, contained in an injection vessel,
(3-2) a first hermetically sealed separation vessel comprising a first port to import the induced antigen-specific cytotoxic T lymphocyte, a second port to drain waste fluid, a third port to inject a separation medium for the antigen-specific cytotoxic T lymphocytes, and a fourth port to export an antigen-specific cytotoxic T lymphocyte after separation,
(3-3) at least two proliferation culture media, each containing IL-2 or IL-15, or both of them in an injection vessel, and
(3-4) a hermetically sealed culture vessel comprising a first port to import an antigen-specific cytotoxic T lymphocyte after separation, a second port to import the prepared antigen-presenting cell, a third port to inject serum or plasma that is separately prepared, a fourth port(s) to inject the proliferation culture medium wherein the number of the ports is at least the same as the number of the proliferation culture media, and a fifth port to export the proliferated antigen-specific cytotoxic T lymphocyte.

13. A method for preparing an antigen-specific cytotoxic T lymphocyte, wherein a preparation kit according to any one of claims 1 to 12 is used.
